# EUROPEAN PATENT APPLICATION

(11) **EP 3 378 950 A1**
(43) Date of publication of application: **26.09.2018**
(21) Application number: 17382141.4
(22) Date of filing: 21.03.2017
(51) Int. Cl.: C12Q 1/68

(54) **EASY ONE-STEP AMPLIFICATION AND LABELING (EOSAL)**

(71) Applicant: Sequencing Multiplex SLK, 46118 Serra (ES); Fund. para la Investigación del Hospital Clínico de la Comunidad Valenciana, Inst. de Investigación Sanitaria Clínico de Valencia, 46010 Valencia (ES)
(72) Inventor: Olivares, Mª Dolores, 46118 SERRA (ES); Ivorra, Carmen, 46118 SERRA (ES); Chaves Martinez, Felipe Javier, 46010 VALENCIA (ES); Blesa Lujan, Sebastian, 46010 VALENCIA (ES)
(74) Representative: ZBM Patents ApS

(57) **Abstract**

The present invention relates to the field of PCR amplification and labeling, and genetic analysis. The present invention allows amplification and labeling of DNA fragments in one amplification reaction and based on the use of at least a pair of primers including a tail at the 5'-end, and a pair of primers comprising the total or partial sequence of one tail, and wherein at least one of the second pair of primers is labeled. The procedure is developed in a single PCR reaction. The invention is also related to kits for nucleic acid amplification, labeling and detection, and to the use of said kits in applications such as genetic diagnosis.

## Description

### FIELD OF THE INVENTION

The present invention relates to the field of genetic diagnosis and genetic analysis of hereditary diseases and, more particularly, to PCR-based methods and kits for genetic analysis.

### BACKGROUND ART

Currently, there are available many PCR-based methods for genetic analysis involving the generation of labeled amplification products. These methods have many different applications, among them: (1) detection of STRs (Short Tandem Repeats); (2) detection and genotyping of genetic polymorphisms by allele specific oligonucleotides (ASOs); (3) detection of large rearrangements; and (4) generation of DNA libraries for New Generation Sequencing (NGS). However, these PCR-based methods for genetic analysis have serious limitations, among them, the need to use a large number of labeled primers (i.e. at least one per amplicon), or the performance of at least two consecutive reactions.

For instance, there are several methods for the detection of large rearrangement available in the art, among them: (1) Southern Blot, karyotyping or fluorescent in situ hybridization (FISH) which are not based on PCR and are limited and time consuming procedures; (2) long PCR, with serious reproducibility problems; (3) real-time quantitative PCR, which implies laborious fragment analysis in each amplification (Barrois et al. 2004 Clin Genet 65(2):131-6); (4) PCR with fluorescently-marked oligos, based on multiplex PCR for several segments, which is very expensive and not very reproducible; and (5) semi-quantitative multiplex PCR (García-García et al. 2006 Human Mutation 27(8): 822-828) consisting of a two PCR consecutive protocol of amplification based on specific amplification of several fragments with tailed primers, and a second PCR reaction for fragment labeling and, finally, fragment analysis in a capillary DNA Sequencer. All of these methods require long procedures, large number of reactions, they are time consuming and expensive.

In summary, the currently available methods for specific DNA amplification and labeling, overall PCR-based protocols are expensive and/or time consuming due to the inclusion of many labeled primers and/or duplication of the number of reactions. In addition, the required manipulation of the amplified products in the second and further PCR steps increments the risk of contamination and errors.

Consequently, there is a clear need to develop PCR-based methods for genetic analysis, which are cheaper, simpler and less time-consuming.

### SUMMARY OF THE INVENTION

In a first aspect, the invention relates to a one-step PCR-based in vitro method for the generation of labeled amplicons from at least a nucleic acid target region in a sample, wherein at least two pairs of PCR primers are used to obtain the labeled amplicons:
- a first pair of PCR amplifying primers comprising a reverse and a forward primer, wherein each PCR amplifying primer comprises at least two regions, a first region or tail, located at the 5'-end, which is not complementary to the at least nucleic acid target region, and a second region, located at the 3'-end, which is sufficiently complementary to the at least nucleic acid target region to allow the amplification of the at least nucleic acid target region to obtain amplicons; and wherein the sequence of the tail of the primers is different from each other; and
- a second pair of PCR labeling primers, wherein one of the labeling primers has a sequence which is sufficiently identical to the tail of one of the amplifying primers, and the other labeling primer has a sequence which is sufficiently identical to the tail of the other amplifying primer, to allow amplification of the amplicons obtained using the first pair of PCR amplifying primes, and wherein at least one of the primers of the second pair of PCR labeling primers is labeled.

In a second aspect, the invention relates to a kit for the above one-step PCR-based in vitro method for the generation of labeled amplicons from at least a nucleic acid target region in a sample, comprising at least two pairs of PCR primers:
- a first pair of PCR amplifying primers comprising a reverse and a forward primer, wherein each PCR amplifying primer comprises at least two regions, a first region or tail, located at the 5'-end, which is not complementary to the at least nucleic acid target region, and a second region, located at the 3'-end, which is sufficiently complementary to the at least nucleic acid target region to allow the amplification of the at least nucleic acid target region to obtain amplicons; and wherein the sequence of the tail of the primers is different from each other; and
- a second pair of PCR labeling primers, wherein one of the labeling primers has a sequence which is sufficiently identical to the tail of one of the amplifying primers, and the other labeling primer has a sequence which is sufficiently identical to the tail of the other amplifying primer, to allow amplification of the amplicons obtained using the first pair of PCR amplifying primes, and wherein at least one of the primers of the second pair of PCR labeling primers is labeled.

And in a third aspect, the invention relates to the use of the kit in the diagnosis of a disease involving at least one or more large rearrangements, small mutations, genetic polymorphisms, CNVs and combinations thereof.

### BRIEF DESCRIPTION OF THE FIGURES

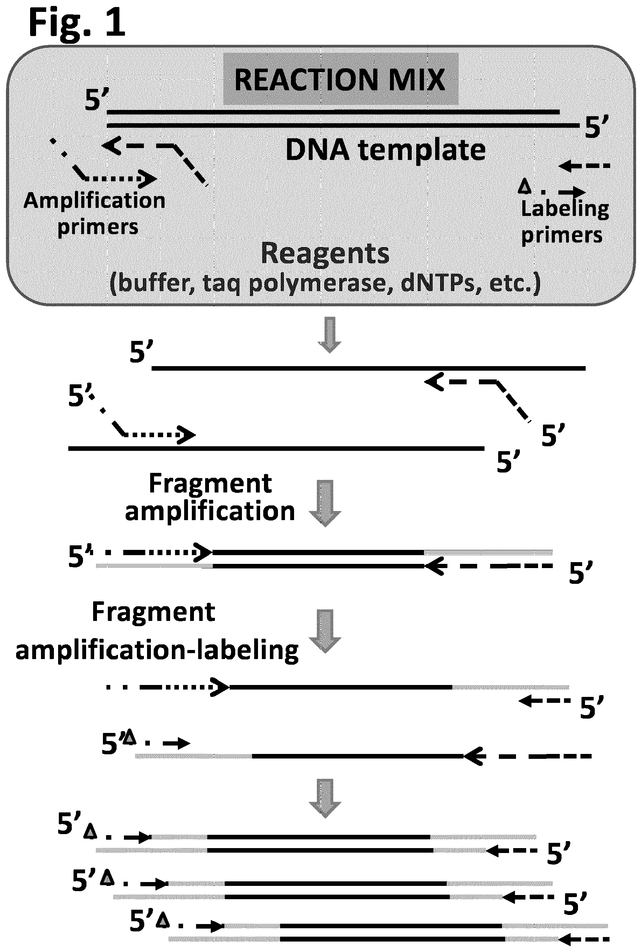

**Fig. 1** shows a scheme of the process of amplification and labeling in one PCR reaction of one PCR product or amplicon, corresponding to a particular embodiment of the present invention. A pair of amplifying primers with different tails (i.e. tail A and B) is used. Additionally, a pair of labeling primers each one comprising the sequence of the tail of the forward or the reverse amplifying primer is also added to the PCR reaction mix. One of the labeling primers is labeled (indicated as a triangle). The amplification procedure starts with the initial amplification of the amplicon by the amplifying primers. PCR cycles generate amplicons including the tails and therefore the labeling primers can amplify the amplicons by the hybridization to the generated complementary sequences of the tails. After several PCR cycles a part or all amplicons are labeled with the labeling primers. The labeled amplicons can be detected, for example, by DNA fragment analysis in a capillary DNA analyzer or sequencer.

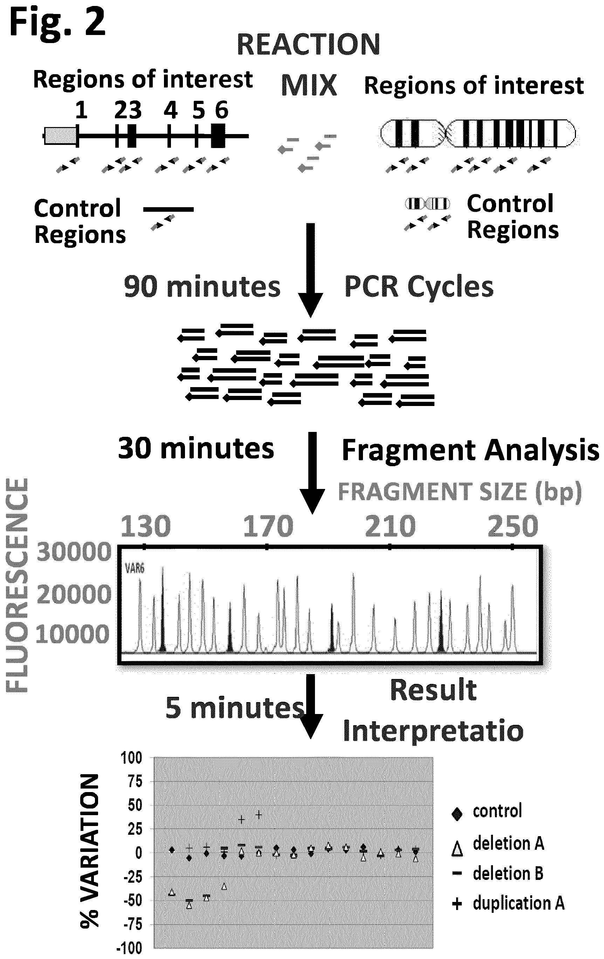

**Fig. 2** shows a scheme of the process for the detection of large rearrangements (including large insertions, large deletions, and large duplications and chromosomal alterations) as well as CNVs, corresponding to a particular embodiment of the present invention. The obtained PCR products are loaded into any system for DNA fragments analysis and quantification, based on the detection of the covalently bound label, involving peak intensity analysis and normalization, graphic representation, calculation of the percentage of variation compared to controls and identification of the arrangement.

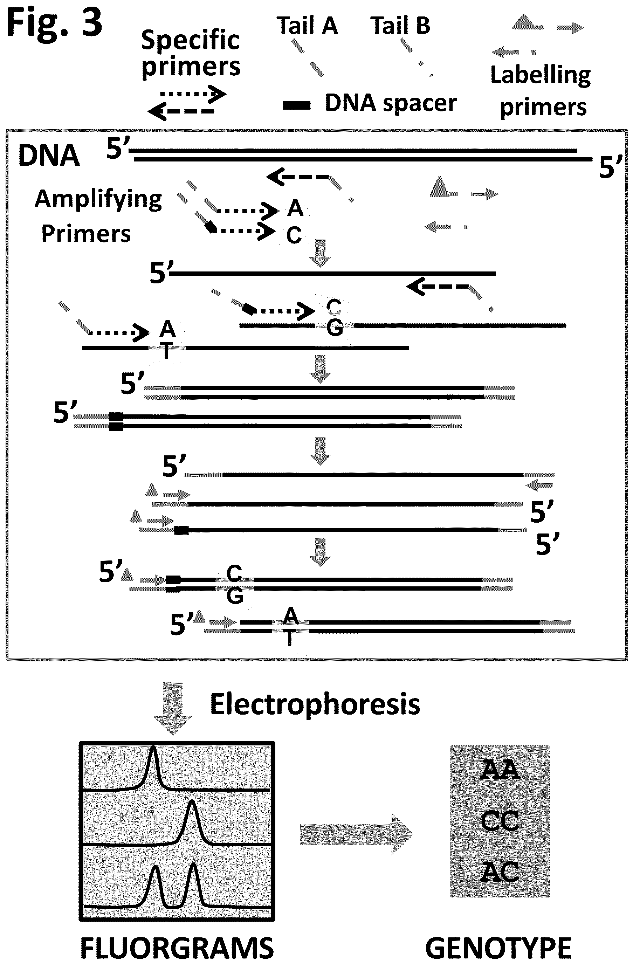

**Fig. 3** shows a schematic representation of the process for the specific amplification and discrimination of the 2 alleles of a SNP or a small mutation by amplicon size, corresponding to a particular embodiment of the present invention. The procedure includes the use of a pair of amplifying primers which are also ASO primers, and wherein each one matches a specific allele of the SNP or the small mutation. The amplifying primers include the tail at the 5'-end but additionally in one of the ASO primers, there is a spacer at the 3'-end of the tail. Each ASO primer allows the amplification of the corresponding allele if it is present in the sample tested. The generated amplicons corresponding to the different alleles are separated by size in a capillary DNA sequencer, since the presence of one allele produces one peak of a specific size, while the presence of 2 alleles produces 2 peaks of specific sizes.

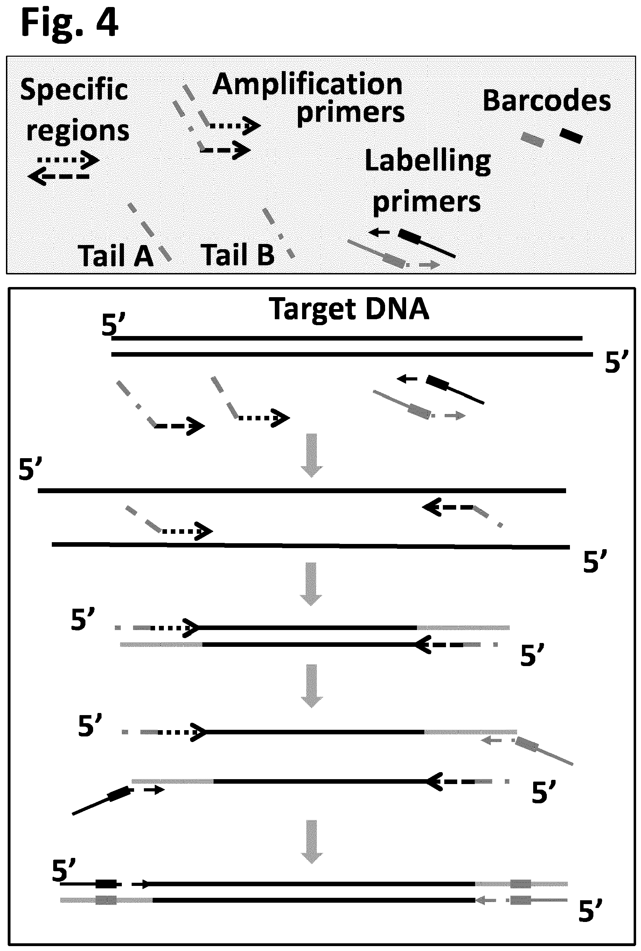

**Fig. 4** shows the schematic representation of the amplification of one genetic region and its labeling for their application in a new generation sequencing system, corresponding to a particular embodiment of the present invention. The procedure includes the use of the labeling primers comprising different sequences, such as barcodes, sequences for hybridization to flow cells and sequences for the specific new generation sequencing system used.

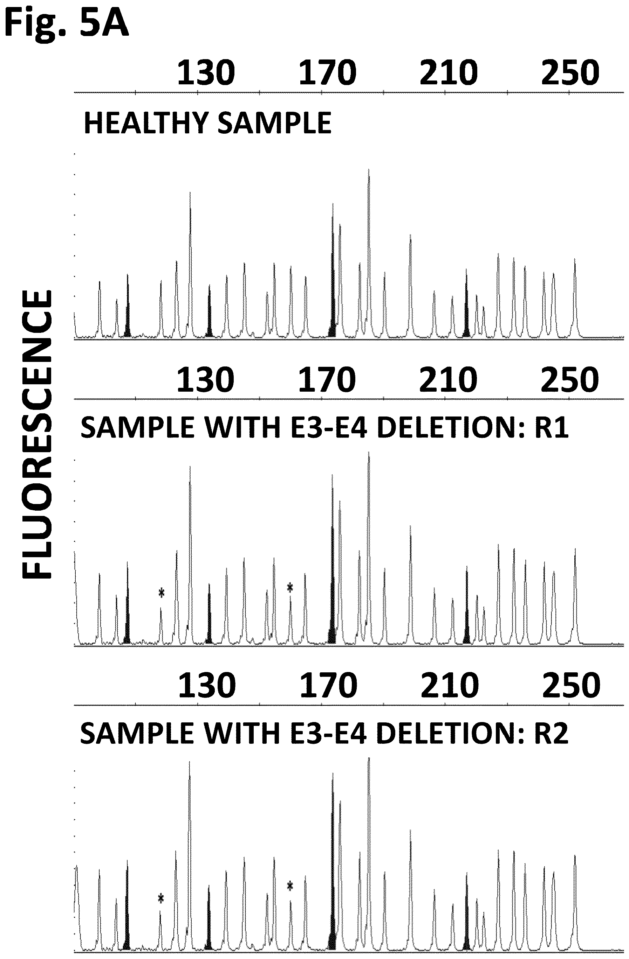

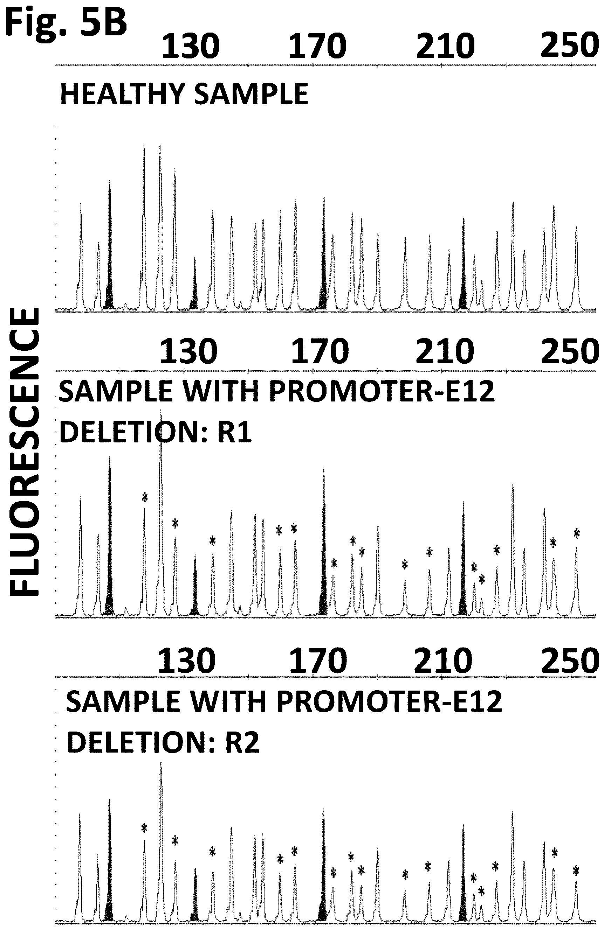

**Fig. 5** corresponds to the electrofluorograms obtained in Example 1 when testing for BRCA1 large rearrangements in a control sample (upper one) and the two replicates of a sample of an affected subject (middle and bottom one, respectively) (FLUO means fluorescence). A. Electrofluorograms obtained in the method of the invention for the sample of a subject with exons 3 and 4 deleted (E3E4); B. Electrofluorograms obtained in the method of the invention for the sample of a subject with a deletion of the promoter and exons 1-12 (PromE12).

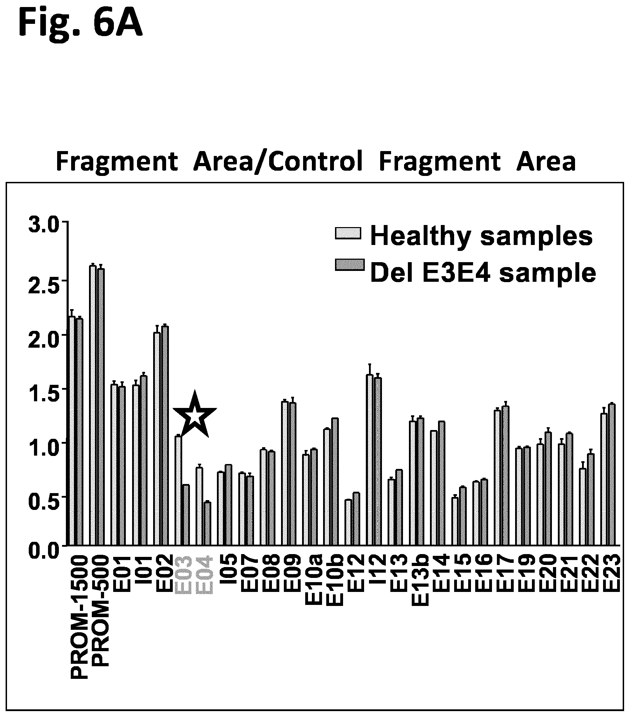

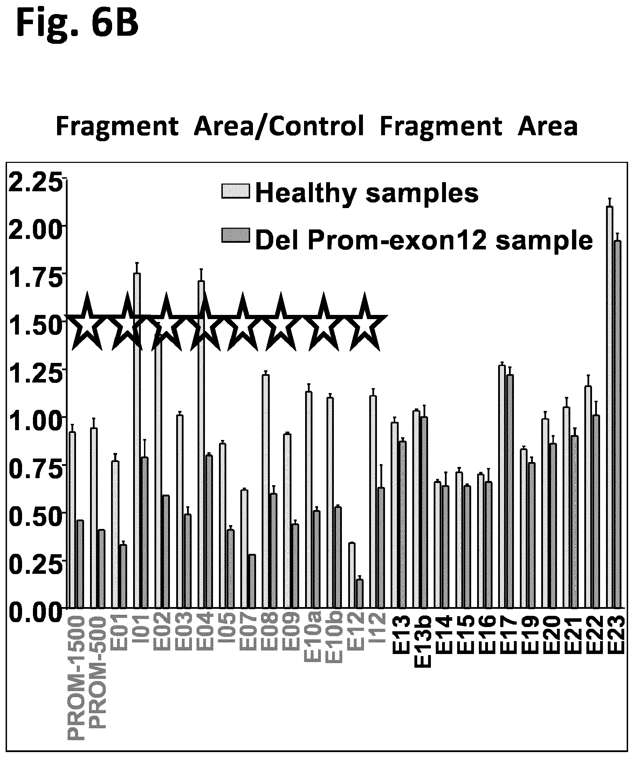

**Fig. 6** shows the variation in the peak-fragment intensity after normalization with control fragments of Example 1. A. Normalized intensities obtained in the method of the invention for the sample of a subject with exons 3 and 4 deleted (E3E4); B. Normalized intensities obtained in the method of the invention for the sample of a subject with a deletion of the promoter and exons 1-12 (PromE12).

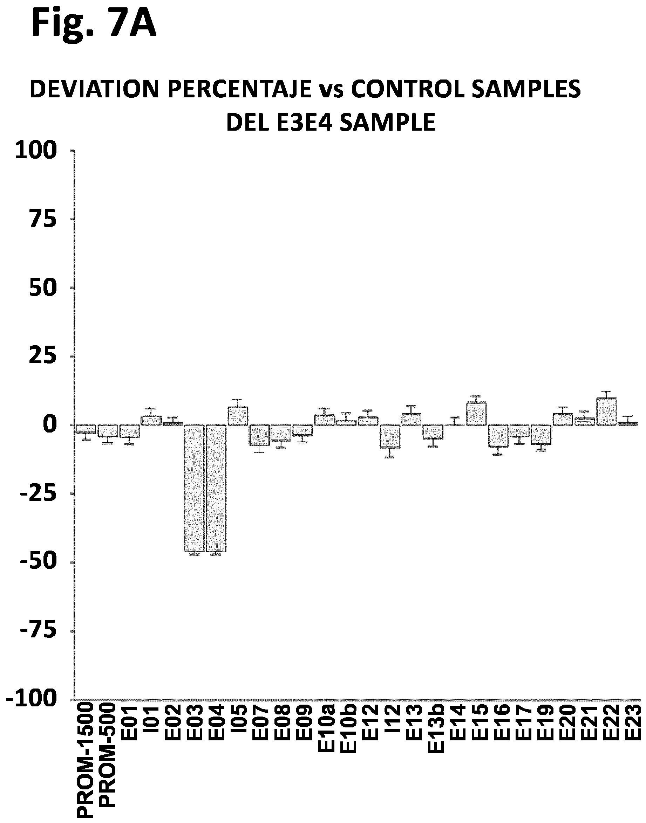

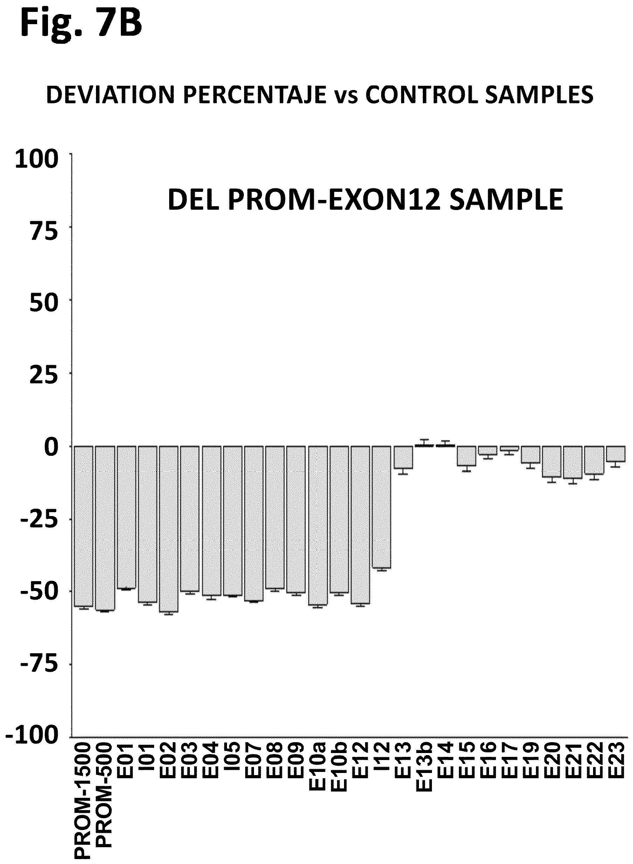

**Fig. 7** shows the changes in the proportion of each of the fragments of the human BRCA1 gene analyzed in each patient with a large rearrangement of Example 1. A. Percentage of deviation obtained in the method of the invention for the sample of a subject with exons 3 and 4 deleted (E3E4); B. Percentage of deviation obtained in the method of the invention for the sample of a subject with a deletion of the promoter and exons 1-12 (PromE12).

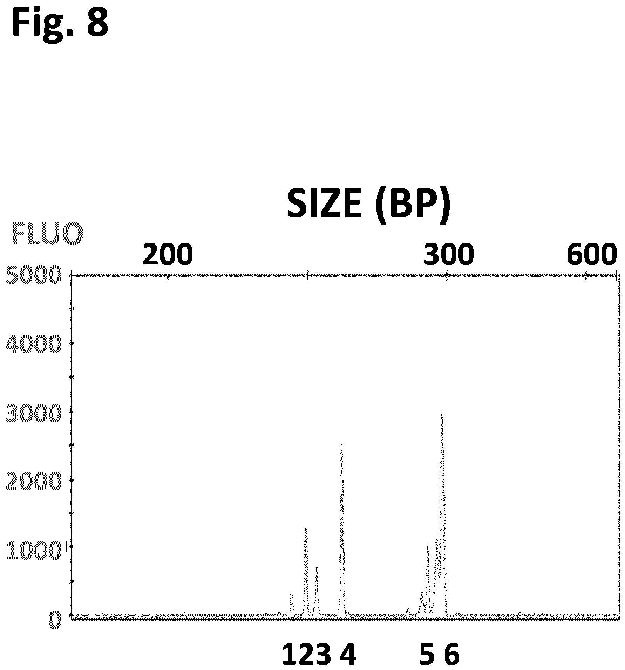

**Fig. 8** is the electrofluorogram obtained in Example 2 (FLUO means fluorescence). Peak 1 represents the results for the rs41525747 SNP of a sample homozygous for the C allele. Peaks 2 and 3 correspond to the rs4988235 SNP of a heterozygous sample. Peak 4 represents the results for the rs41380347 SNP of a sample homozygous for the T allele. Peaks 5 and 6 correspond to the rs182549A SNP of a heterozygous sample.

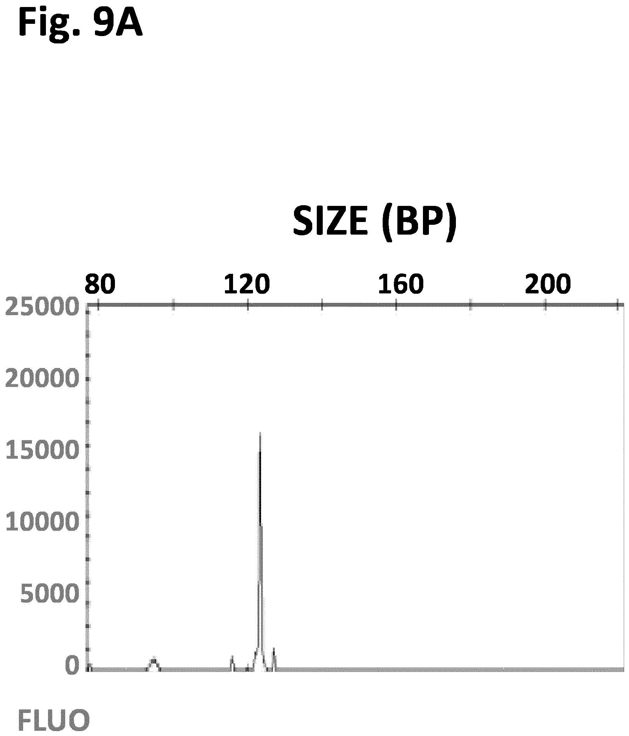

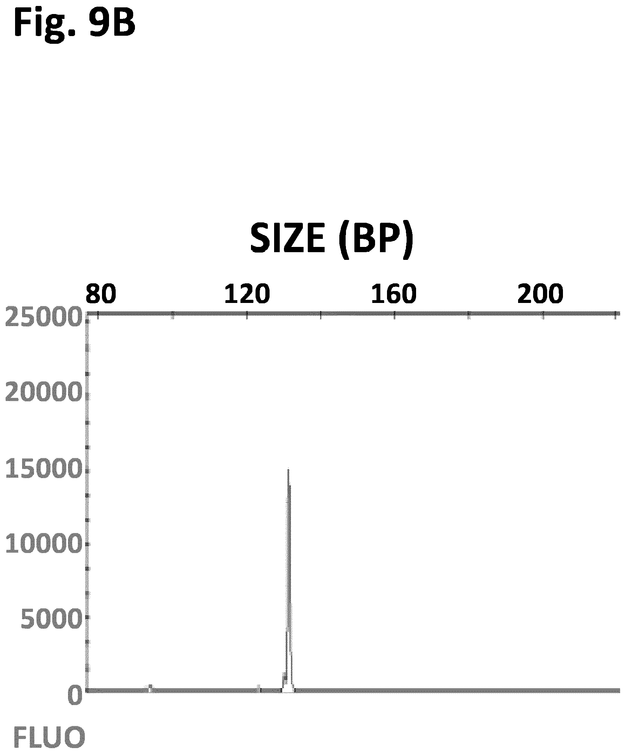

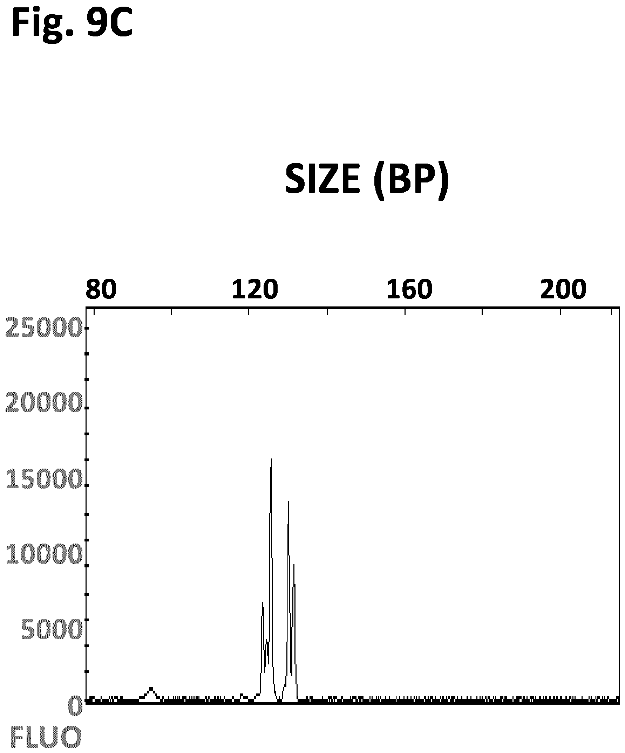

**Fig. 9** shows the identification HLA-DQA1 haplotypes. A. homozygous individuals for DQA1*01; B. homozygous for DQA1*03; C. heterozygous for both haplotypes (FLUO means fluorescence).

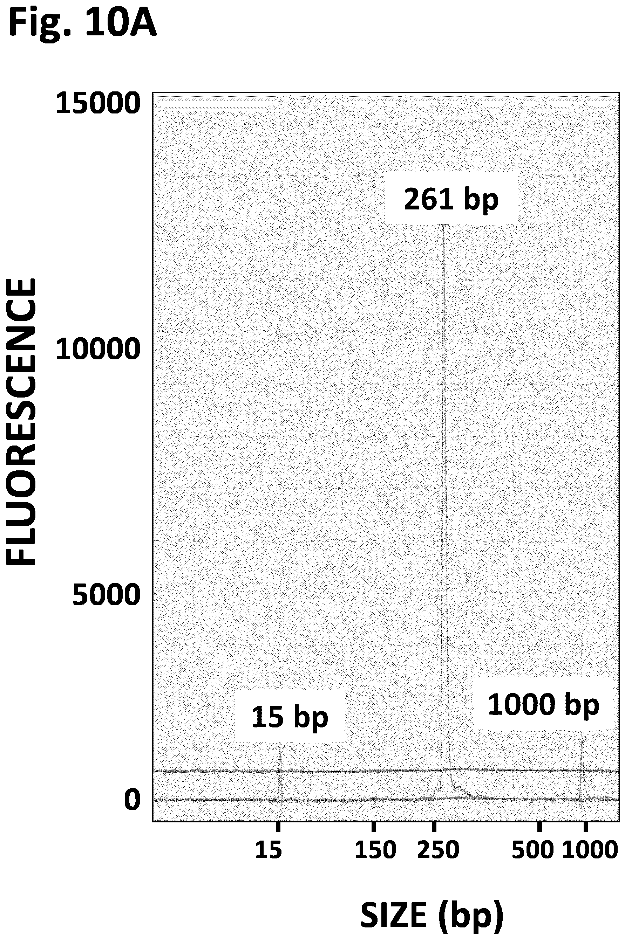

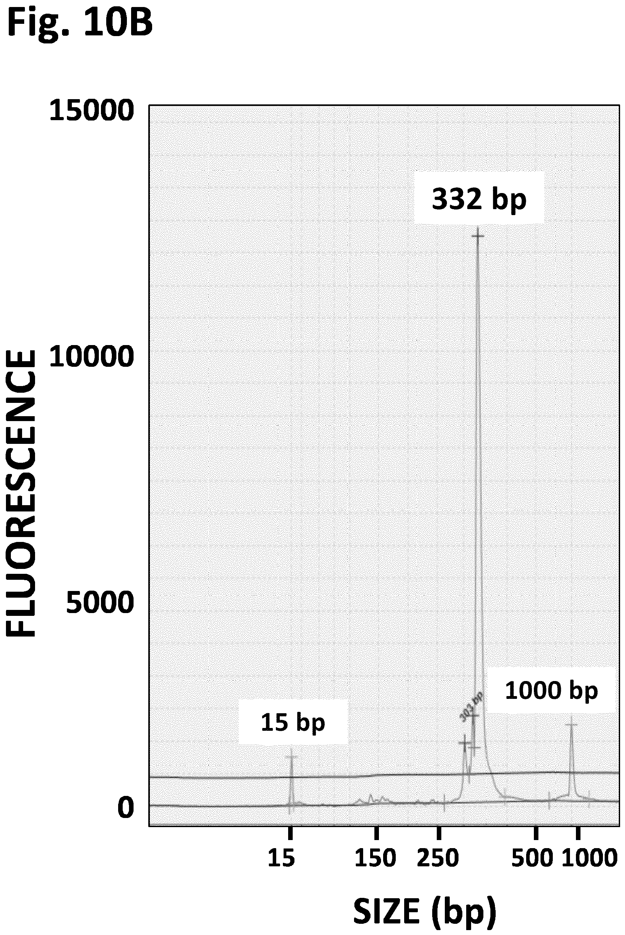

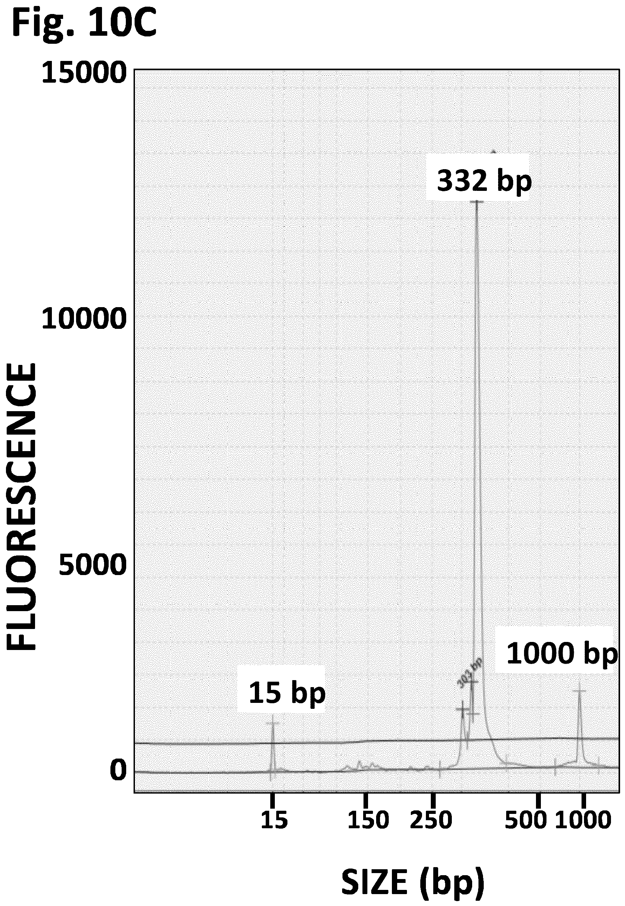

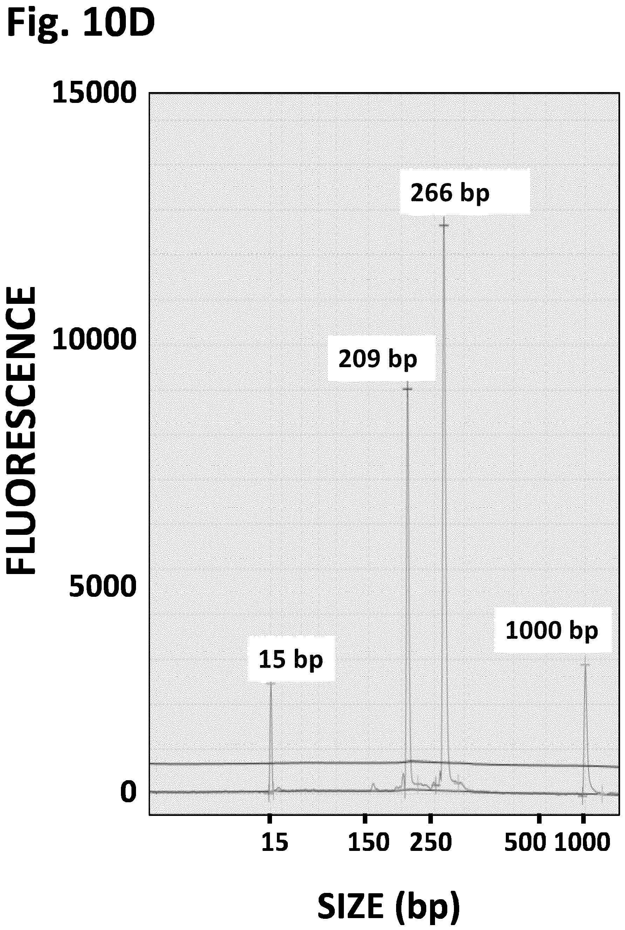

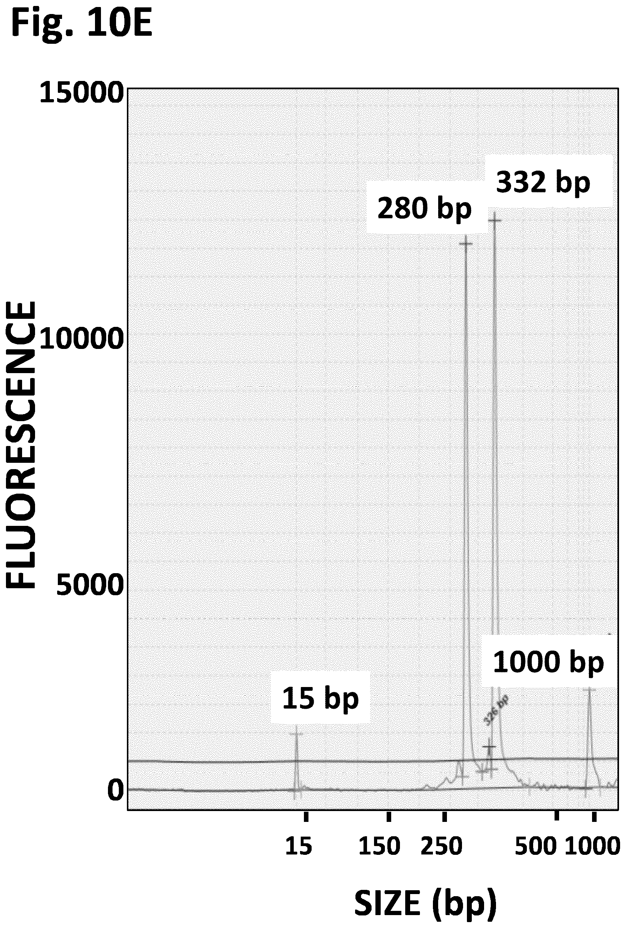

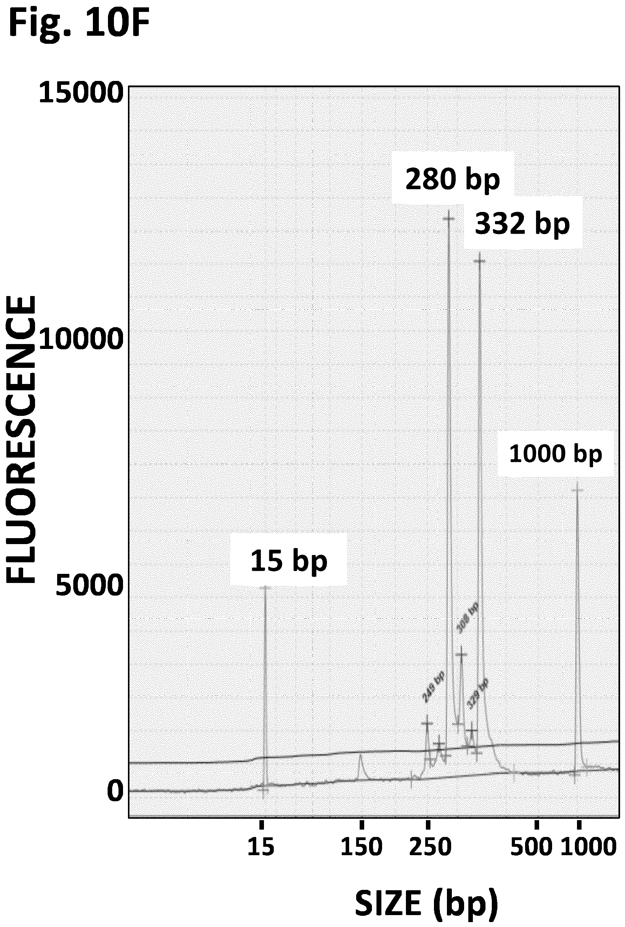

**Fig. 10** shows the electrofluorograms corresponding to exons 2 and 3 of the human KRAS gene of Example 4 (FLUO means fluorescence).

### DETAILED DESCRIPTION OF THE INVENTION

### Definitions

The terms "primer", "oligonucleotide" and "oligo" are used herein indistinctly, and refer to an oligonucleotide that acts to initiate synthesis of a complementary nucleic acid strand when placed under conditions in which synthesis of DNA by primer extension is induced, e.g., in the presence of nucleotides and a DNA polymerase, at suitable temperature, pH, metal ion concentration, and salt concentration, etc.

The terms "5'-end" and "3'-end" are used herein to indicate the extremes of a strand of a nucleic acid. The term "5'-end" relates to the end of a nucleic acid strand that has the fifth carbon in the sugar-ring of the deoxyribose or ribose at its terminus. The term "3'-end" relates to the end of a nucleic acid strand that has a hydroxyl group at the third carbon of the sugar ring. All sequences are indicated in the direction 5'-end to 3'-end.

The term "tail" refers to a nucleotide sequence between approximately 10 to 100 nucleotides (nt), preferably, between 10-80 nt, more preferably, between 10-40 nt, and even more preferably between 10-30 nt, that does not hybridize with a target DNA, and is located in the 5'-end of the portion of a primer that hybridizes with the target DNA.

The term "DNA labeling" refers to the inclusion of anything that allows the identification of a DNA molecule by any suitable technology known in the state of the art. Usually it includes covalently bound molecules such as fluorophores, radioactive molecules, molecules such as biotin or digoxig-enin, and reactive groups (such as phosphate, amines, etc.). Another way of DNA labeling is the inclusion of RNA or DNA sequences using normal or modified nucleotides (also known as nucleotide sequence label, or analogs such as peptide nucleic acids) allowing the identification of the DNA, such as in NGS.

The terms "amplicon", "PCR product", "amplification product", "amplified product" and "amplified fragment" are used indistinctly to refer to a genetic region (piece of DNA or RNA) that is the product of artificial amplification using specific primers for its amplification.

The term "large rearrangement" refers to a change in the normal arrangement of the genome. It usually occurs as a consequence of double-strand breaks of the DNA, followed by abnormal rejoining of the non-homologous ends. Alternatively, a chromosome rearrangement can result from crossing-over between repetitive DNA sequences. This term applies to those changes involving at least 100 bp, and in many cases can be visible cytogenetically, resulting in "cytogenetic abnormalities". Large rearrangements include, but are not limited to "large deletions", "large duplications" and "large insertions". A special kind of large rearrangement can be considered the duplication or elimination of a complete chromosome.

The term "deletion" refers to a type of mutation caused by loss of one or more nucleotides from a DNA segment. Deletions can be large, known in the context of the present invention as "large deletions", encompassing a part of a gene, many genes and megabases of DNA, to the point of producing a visible cytological abnormality in a chromosome. A special large deletion can be considered the absence of one chromosome. Or it may be limited to one or a few base pairs, in general up to 100 bp (known in the context of the present invention as "small deletions").

The term "duplication" relates to an additional copy of a DNA segment present in the genome. Duplications lead to an increase in the number of copies of one DNA segment that can up to 100 bp ("small duplication"), or 100 bp or more ("large duplication"). Large duplications can include a fragment of one gene, complete genes or a large part of a chromosome and it may or may not be cytogenetically visible. A special duplication can be considered the inclusion of an extra copy of a chromosome.

The term "insertion" refers to a type of mutation in which one or more nucleotides are inserted into a DNA sequence. A "large insertion" in the context of the present invention indicates an insertion of more than 100 bp, eventually resulting in the introduction of a genome region in another location producing a partial duplication of a gene or chromosomal region. On the contrary, an insertion may be limited to one or a few base pairs, in general up to 100 bp (known in the context of the present invention as "small insertion").

The term "genetic polymorphism" refers to the occurrence in the same population of two or more alleles at one locus, each with appreciable frequency, where the minimum frequency is typically taken as 1%.

The term "allele" refers to each one of the two or more forms of a genetic polymorphism. Most multicellular organisms have two sets of chromosomes, that is, they are diploid, except for specific genes usually present in sexual chromosomes. If both alleles of a polymorphism are identical, the organism is homozygote for it. On the contrary, ilf the alleles are different, the organism is heterozygote.

The term "copy number variation" (often abbreviated to CNV) is referred to a particular type of genetic polymorphism characterized by an abnormal number of copies of one or more sections of the DNA. This term comprises both deletion (also known as "reduced CNV") and duplication (also known as "amplified CNV") of relatively large genome regions on certain chromosome regions. Each copy number variation may range from about 100 bp to several megabases in size.

The term "single nucleotide polymorphism" (often abbreviated to SNP) is referred to a particular type of genetic polymorphism, namely a variation in a single nucleotide that occurs at a specific position in the genome.

The term "haplotype" is referred to an individual collection of specific alleles of genetic polymorphisms and/or mutations within a given genetic segment of a DNA molecule.

The term "small mutation" refers to a type of mutation in a genomic region including up to 100 bp. The small mutation may be a "small substitution", "small insertion", "small deletion" or "small duplication".

The terms "New Generation Sequencing" and "Next Generation Sequencing" (often abbreviated to NGS), also known as high-throughput sequencing, refer to the catch-all terms used to describe a number of different sequencing technologies, including without limitation, Sequencing by Synthesis (SBS) from Illumina, Pyrosequencing from Roche, Ion Torrent™ semiconductor sequencing technology and Sequencing by Oligo Ligation Detection (SOLiD) by Applied Biosystems. All of them allow the simultaneous sequencing of thousands to millions of DNA fragments.

The term "allele specific oligonucleotide" (often abbreviated to ASO) refers to a primer complementary to the sequence of a target DNA containing an allele of a SNP or a small mutation. An ASO is typically an oligonucleotide of approximately between 10 and 40 nt in length, preferably between 15 and 25 nt, designed (and used) in a way that makes it specific for only one allele of the tested DNA.

The term "barcode" refers to a known nucleotide sequence included in a primer sequence to allow its identification. The barcodes are usually used in NGS for sample identification of the sequence data.

The term "nucleotide sequence label" refers to any DNA sequence used as a label. In NGS the nucleotide sequence label includes different nucleotide sequences, including without limitation, the barcode, the sequence used for flow cell hybridization, the sequence for sequencing primer hybridization, etc.

The terms "nucleotide spacer" and "spacer" are used indistinctly to refer to a short nucleotide sequence between approximately 1 and 100 nt, preferably between 1 and 50 nt, more preferably between 1 and 20 nt, and even more preferably between 1 and 10 nt, incorporated in a primer between the tail at the 5'-end and the nucleotide sequence hybridizing to the target DNA.

A nucleic acid molecule is said to be "complementary" with another nucleic acid molecule if the two molecules share a sufficient number of complementary nucleotides to form a stable duplex when the strands bind (hybridize) to each other under the required conditions. Complementarity is conveniently described by percentage, that is, the proportion of nucleotides that form base pairs between two molecules or within a specific region or domain of two molecules. The term "sufficient complementarity" means that a sufficient number of base pairs exist between one nucleic acid molecule or region thereof and a target nucleic acid sequence to achieve detectable binding or can be used as starting point for amplification (e.g. if it is at least 30%, at least 40%, at least 50%, at least 60%, at least 70%, at least 80%, or at least 90%).

The term "size reference value" refers to the size of an amplicon based on a reference sequence of normal genome (i.e. in the absence of an insertion, duplication, small mutation, etc.) available in public database, such as Ensembl.

The expression "quantity reference value" refers to the quantity of an amplicon based on a reference sequence of a normal genome (i.e. in the absence of large duplication, trisomy, etc.) available in public databases, such as Ensembl.

The authors of the present invention have developed a method that allows producing from one to thousands of different types of labeled amplification products using a reduced number of labeled primers per reaction, and performing both, the amplification and the labeling of the amplified products, in a single reaction step. Current methods require one labeled primer in each primer pair used in the PCR reaction for labeling each amplicon or two separate reactions to obtain labeled amplified products. The method of the present invention is much easier to prepare which means a very significant reduction in (i) the amount of time and labor necessary to prepare it; (ii) the waiting time to receive the results; and especially, (iii) the costs of the method (i.e. labeled primers are usually over 10 times more expensive than non-labeled ones). For instance, with standard procedures for labeling 100 amplicons in 100 samples, 100 labeled and 100 non-labeled primers are required as well as 100 PCR reactions, or alternatively, the use of 201 non-labeled and one labeled primers, and 200 PCR reactions is needed. The present invention requires 201 non-labeled oligos and one labeled but only 100 PCR reactions.

Thus, in a first aspect, the invention is related to a one-step PCR-based *in vitro* method for the generation of labeled amplicons from at least a nucleic acid target region in a sample, wherein at least two pairs of PCR primers are used to obtain the labeled amplicons: (i) a first pair of PCR amplifying primers comprising a reverse and a forward primer, wherein each PCR amplifying primer comprises at least two regions, a first region or tail, located at the 5'-end, which is not complementary to the at least nucleic acid target region, and a second region, located at the 3'-end, which is sufficiently complementary to the at least nucleic acid target region to allow the amplification of the at least nucleic acid target region to obtain amplicons; and wherein the sequence of the tail of the primers is different from each other; and (ii) a second pair of PCR labeling primers, wherein one of the labeling primers has a sequence which is sufficiently identical to the tail of one of the amplifying primers, and the other labeling primer has a sequence which is sufficiently identical to the tail of the other amplifying primer, to allow amplification of the amplicons obtained using the first pair of PCR amplifying primes, and wherein at least one of the primers of the second pair of PCR labeling primers is labeled.

In other words, each primer of the pair of PCR amplifying primers is designed to comprise a tail at the 5'-end which does not hybridize with the at least nucleic acid target region of the sample. In an embodiment, the sequences of the tails of the forward and reverse PCR amplifying primers are the same. In an alternative embodiment, the sequences of the tails of the forward and reverse PCR amplifying primers are different between them. The tails allow the further amplification and labeling of the amplicons obtained using the pair of PCR amplifying primers. The tails may vary both in sequence and size depending on the particular embodiment of the method of the invention. The skilled person knows how to design the tails depending on the specific application of the present invention.

The forward and reverse labeling primer may consist of or comprise the sequence of the tail of the forward and reverse amplifying primer, respectively. The sequence of the tail of each PCR labeling primer can be completely or partially identical to the corresponding tail sequence of the PCR amplifying primers, but in any case, to allow the amplification reaction.

Any label available in the art to detect a nucleic acid can be used in the PCR labeling primers used in the method of the present invention. In a particular embodiment, both labeling primers are labeled, using either the same label or different labels. In another particular embodiment, only one of the PCR labeling primers is labeled. The label used may be, without limitation, a label selected from the group consisting of: fluorescent label, chemical label, radioactive label, and nucleotide sequence label located at the 5'-end. The label(s) of the PCR labeling primers except for the nucleotide sequence label, can be in any nucleotide position along the primer(s). In a particular embodiment, the label is located at the 5'-end. When nucleotide sequence labels are used in both PCR labeling primers, the nucleotide sequence labels used may have the same or different sequence. Any type of nucleotide (natural or artificial) can be used.

One or more of the PCR amplifying and/or labeling primers used in any of the embodiments of the present invention may comprise a nucleotide spacer at the 3'-end of their tail. The spacer is used to identify the different amplicons obtained by their size. The skilled person knows how to design the spacer(s), if needed. In a particular embodiment, the spacer has between 1 and 50 nt long, between 1 and 30 nt long, or between 5 and 10 nt long.

The method of any of the embodiments of the present invention may occur in a single reaction, which comprises one experimental condition of amplification cycles. Alternatively, it may be performed using several experimental conditions of amplification cycles.

Similarly, the method may be carried out in any type of sample. In a particular embodiment, the sample is selected from the group consisting of human, animal, plant, fungal, bacterial, viral and synthetic sample.

The advantages of the method of the present invention allow its use in different applications such as detection of large genetic rearrangements and CNVs, genotyping of point mutations, SNPs and generating NGS libraries with reduced costs, hand work and time required to get the labeled amplicons. And moreover, it can be used for analyzing at the same time polymorphism, large rearrangements and/or CNVs.

The method developed by the inventors, when applied to the detection of large rearrangements, such as large deletions, large amplifications or large insertions, has the great advantage of being much more simple, rapid and easy than the current MLPA technique, since it allows the detection of the large rearrangement in a few hours and with a minimum work (i.e. two pipettings steps, one for the PCR reaction and one for the Genetic Analyzer loading). Additionally, the method of the present invention can be used in the detection of large rearrangements in homozygosis as well as in heterozygosis.

The present invention allows the detection of large rearrangements by only one PCR reaction. The labeled amplicons obtained in the method of the present invention may be quantified and/or sized. If the size of the labeled amplicons is that of a size reference value and if the quantity of the labeled amplicons is increased when compared to a quantity reference value, then it is indicative of an amplified copy number variation (CNV); and if the size of the labeled amplicons is that of a size reference value and the quantity of the labeled amplicons is decreased when compared to a quantity reference value, then it is indicative of a reduced CNV.

The calculations comprise the normalization of each tested amplicons to the control amplicons in control and tested samples. These data are used to know the percentage of variation in the intensity (measured either as height or area) of each amplicon peak in tested samples in relation to normal samples. If there is a reduction or increase over approximately 25-30%, the data indicate the presence of a deletion or insertion, respectively. In the case of amplification, an increase of approximately 40-50% indicates an increase in one extra copy.

In a particular embodiment of the present invention, the method allows is used for the detection of one or more haplotypes, each of them composed of several polymorphisms. In this embodiment, at least one of the primers of the pair of PCR amplifying primers contains at different nucleotide positions, several alleles to determine the haplotype. In other words, a forward PCR amplifying primer is used for each haplotype, the forward PCR amplifying primer comprises at the 3'-end the specific combination of alleles of the haplotype to be genotyped and a tail at its 5'-end. A reverse PCR amplifying primer is also used which comprises a tail at its 5'-end. In a particular embodiment, the reverse PCR amplifying primer also contains at different nucleotide positions, different alleles of the haplotype to be genotyped. All amplifying PCR forward primers contain the same tail sequence. A pair of PCR labeling primers is also used, wherein the forward and reverse PCR labeling primer comprises the tail of the forward and reverse PCR amplifying primer, respectively, and wherein at least one of them is labeled. The identification of each haplotype is achieved by the different size of the amplified products. Alternatively, different labels can be used to detect each haplotype. Alternatively, a combination of spacers and different labels can be used to detect the haplotypes. In another particular embodiment of the present invention, the method is used for allele genotyping wherein at least one of the primers of the first pair of PCR amplifying primers is an ASO primer. In both cases, the labeled amplicons are sized, so that if the size is that of a size reference value, then it is indicative of the presence of the haplotype or allele, respectively, to be determined.

The present invention can be used for the detection of each allele of one or several SNPs or of small mutations. Previous methodology requires that at least one of the primers used for the detection of each SNP must be labeled. The method of the present invention requires only one labeled primer for the detection of several SNPs. Usually, two forward PCR amplifying primers are used for each SNP, wherein both of said primers are ASO primers with a tail at the 5'-end. A reverse primer is also added to the reaction. In a particular embodiment, a spacer sequence at the 3'-end of the tail may be introduced when needed so that the final amplification products of each allele of the genotyped SNPs have different sizes. A pair of PCR labeling primers is also included in the PCR reaction, wherein only one of the pair of PCR labeling primers is labeled. In the case of the genotyping of the SNP, if the size of the amplicon is that of a reference size value, the specific allele of the ASO primer is determined. In the case of the detection of small mutations, the labeled amplicons are sized, so that if the size of the labeled amplicons is increased in less than 100 bp, when compared to a size reference value, then it is indicative of a small insertion, and if the size is decreased in less than 100 bp, when compared to a size reference value, then it is indicative of a small deletion; if the increase or decrease is 100 bp or more, then it is indicative of a large insertion or large deletion, respectively.

In another particular embodiment, the method of the present invention is used with a NGS technology. In this case, the sequence of the amplicons is determined, and DNA labels, such as the barcodes and all DNA sequences required in DNA libraries used in NGS are included in the produced labeled amplicons. These DNA labels are necessary for NGS, wherein each type of NGS technology, such as, without limitation, Roche, Illumina or Thermo Fisher, requires specific DNA labels (or DNA sequences). In the case of libraries of amplicons, there are different procedures described and used, but all of them require the performance of several steps and usually two consecutive PCR steps, the first one is used for the amplification of the regions of interest. And. the second step is used for the barcoding of the amplicons by amplification in PCR. The method of the present invention allows the specific amplification of different regions or amplicons and their labeling in only one step in order to proceed with the NGS reactions.

In a particular embodiment, the at least nucleic acid target region detected is located in a chromosomal region selected from the group consisting of: -6q21, -13q14.3/13q34, +12, 14q32.3, Amp 8q24.1 (MYC), Amp 3q27.3-q28, +X, Xp, -6q23.2-q25, -6q13-15, Amp Bcl-2 (18q21), +3, -7q32, - 14q, +18q21, Amp 3q27-29, - 8p21-pter, -9p21-pter,- 9q21-q32, Amp Bcl-6 (3q27), Amp Bcl-2 (18q21), Amp Myc (8q24.1), 14q32.3, amp(1q21) (CKS1B), 1p32.3, -13q14, -17p13, -3q, -5q, - 7/7q-, +8, -12p, del 13q, -20q, +19, i17q, -10q23.31 (PTEN), -11q22.3 (ATM), and -17q (TP53).

In another particular embodiment, the at least nucleic acid target region is a region of a gene selected from the group consisting of ABCA1, ABCB11, ABCG5, ABCG8, AKT, ALK, ANK2, APC, APOA1, APOB, APOC2, APOE, APP, ARH, ASXL1, ATM, ATP8B1, BIRC3, BRCA1, BRCA2, CBS, CEBPA, CETP, CFTR, CKIT, CLCNKB, CLDN16, CLDN19, COL1A1, COL1A2, COL3A1, CYP21A2, DAX1, DMD, DMGDH, DNMT3A, EFGFR, EGR2, EPCAM, ERBB2, ERBB3, ERS1, FBN1, FBXW7, FGFR1, FGFR2, FGFR3, FGFR4, FHF6, FLCN, FLT3-ITD, FM01, FM03, FRAF, GCK, GNA11 , GNAQ, HNF1A, HNF1B, HNF4A, HRAS, IDH1, IDH2, KCNE1, KCNE2, KCNH2, KCNJ2, KCNQ1, KEAP1, LCAT, LDLR, LPL, MEFV, MEK1, MEN1, MEN2, MLH1, MSH2, MTHFR, MTP, MYD88, NF1, NF1, NF2, NFKBIE, NOTCH1, NRAS, NRAS, PAX8, PCSK9, PDGFRA, PIK3CA, PKP2, POT1, PRKAR1A, PRSS1, PSEN1, PTEN, RET, RET, RICTOR, ROS1, RUNX1, RUNX1, SCN5A, SDHB, SDHC, SDHD, SERPINA1, SF3B1, SLC12A3, SLC22A1, SLC34A2, SMAD4, SMN1, SMO, SPINK1, STK11, TET2, TGFBR1, TGFBR2, TP53 and XPO1 genes.

### Kits of the invention

In an additional aspect, the invention relates to a kit for one-step PCR-based *in vitro* method for the generation of labeled amplicons from at least a nucleic acid target region in a sample, comprising at least two pairs of PCR primers: (i) a first pair of PCR amplifying primers comprising a reverse and a forward primer, wherein each PCR amplifying primer comprises at least two regions, a first region or tail, located at the 5'-end, which is not complementary to the at least nucleic acid target region, and a second region, located at the 3'-end, which is sufficiently complementary to the nucleic acid target region to allow the amplification of the at least nucleic acid target region to obtain amplicons; and wherein the sequence of the tail of the primers is different from each other; and (ii) a second pair of PCR labeling primers, wherein one of the labeling primers has a sequence which is sufficiently identical to the tail of one of the amplifying primers, and the other labeling primer has a sequence which is sufficiently identical to the tail of the other amplifying primer, to allow amplification of the amplicons obtained using the first pair of PCR amplifying primes, and wherein at least one of the primers of the second pair of PCR labeling primers is labeled.

In a particular embodiment, at least one of the primers of PCR labeling primers of the kit of the present invention is labeled with a label selected from the group consisting of: fluorescent label, chemical label, radioactive label, and nucleotide sequence label located at the 5'-end. In another particular embodiment, each primer of the second pair of PCR labeling primers has a nucleotide sequence label located at the 5'-end. In another particular embodiment, only one of the second pair of PCR labeling primers is labeled. In another particular embodiment, at least one primer of the first pair of PCR amplifying primers comprises a nucleotide spacer at the 3'-end of the tail. In still another particular embodiment, the primers amplify a region of a gene selected from the group consisting of the ABCA1, ABCB11, ABCG5, ABCG8, AKT, ALK, ANK2, APC, APOA1, APOB, APOC2, APOE, APP, ARH, ASXL1, ATM, ATP8B1, BIRC3, BRCA1, BRCA2, CBS, CEBPA, CETP, CFTR, CKIT, CLCNKB, CLDN16, CLDN19, COL1A1, COL1A2, COL3A1, CYP21A2, DAX1, DMD, DMGDH, DNMT3A, EFGFR, EGR2, EPCAM, ERBB2, ERBB3, ERS1, FBN1, FBXW7, FGFR1, FGFR2, FGFR3, FGFR4, FHF6, FLCN, FLT3-ITD, FM01, FM03, FRAF, GCK, GNA11 , GNAQ, HNF1A, HNF1B, HNF4A, HRAS, IDH1, IDH2, KCNE1, KCNE2, KCNH2, KCNJ2, KCNQ1, KEAP1, LCAT, LDLR, LPL, MEFV, MEK1, MEN1, MEN2, MLH1, MSH2, MTHFR, MTP, MYD88, NF1, NF1, NF2, NFKBIE, NOTCH1, NRAS, NRAS, PAX8, PCSK9, PDGFRA, PIK3CA, PKP2, POT1, PRKAR1A, PRSS1, PSEN1, PTEN, RET, RET, RICTOR, ROS1, RUNX1, RUNX1, SCN5A, SDHB, SDHC, SDHD, SERPINA1, SF3B1, SLC12A3, SLC22A1, SLC34A2, SMAD4, SMN1, SMO, SPINK1, STK11, TET2, TGFBR1, TGFBR2, TP53, and XPO1 genes.

Suitable kits include various reagents for use in accordance with the present invention in suitable containers and packaging materials, including tubes, vials, controls, standards and shrink-wrapped and blow-molded packages. Additionally, the kits of the invention can contain instructions for the simultaneous, sequential or separate use of the different reagents, which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

### Uses of the invention

In another aspect, the present invention relates to the use of the kit as previously described in the diagnosis of a disease involving one or more large rearrangements, small mutations, genetic polymorphisms, CNVs and combinations thereof. In another particular embodiment, the disease is selected from the group consisting of familial hypercholesterolemia, breast cancer and ovarian cancer. In still another particular embodiment, the invention relates to the use of the kit as previously described, wherein the at least nucleic acid target region is a region of a gene selected from the group consisting of ABCA1, ABCB11, ABCG5, ABCG8, AKT, ALK, ANK2, APC, APOA1, APOB, APOC2, APOE, APP, ARH, ASXL1, ATM, ATP8B1, BIRC3, BRCA1, BRCA2, CBS, CEBPA, CETP, CFTR, CKIT, CLCNKB, CLDN16, CLDN19, COL1A1, COL1A2, COL3A1, CYP21A2, DAX1, DMD, DMGDH, DNMT3A, EFGFR, EGR2, EPCAM, ERBB2, ERBB3, ERS1, FBN1, FBXW7, FGFR1, FGFR2, FGFR3, FGFR4, FHF6, FLCN, FLT3-ITD, FM01, FM03, FRAF, GCK, GNA11 , GNAQ, HNF1A, HNF1B, HNF4A, HRAS, IDH1, IDH2, KCNE1, KCNE2, KCNH2, KCNJ2, KCNQ1, KEAP1, LCAT, LDLR, LPL, MEFV, MEK1, MEN1, MEN2, MLH1, MSH2, MTHFR, MTP, MYD88, NF1, NF1, NF2, NFKBIE, NOTCH1, NRAS, NRAS, PAX8, PCSK9, PDGFRA, PIK3CA, PKP2, POT1, PRKAR1A, PRSS1, PSEN1, PTEN, RET, RET, RICTOR, ROS1, RUNX1, RUNX1, SCN5A, SDHB, SDHC, SDHD, SERPINA1, SF3B1, SLC12A3, SLC22A1, SLC34A2, SMAD4, SMN1, SMO, SPINK1, STK11, TET2, TGFBR1, TGFBR2, TP53, and XPO1 genes.

In another particular embodiment, the kit is used to generate NGS libraries.

An additional aspect of the present invention relates to the NGS library generated using any of the kits of the present invention.

The particulars of the kits according to the invention have been described in detail in the context of the kits of the invention and are applied with same meaning in the context of the uses of said kits.

All terms as used herein, unless otherwise stated, shall be understood in their ordinary meaning as known in the art. Other more specific definitions for certain terms as used in the present application, are as set forth above, and are intended to apply uniformly throughout the description and claims unless an otherwise expressly set out definition provides a broader definition. Throughout the description and claims the word "comprise", and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. Furthermore, the present invention covers all possible combinations of particular and particular embodiments described herein.

The invention is described in detail below by means of the following examples, which are to be construed as merely illustrative and not limitative of the scope of the invention.

### EXAMPLES

### Example 1. Detection of large rearrangement in the human BRCA1 gene

### PCR primers

Thirty amplifying primers pairs, each comprising a forward and a reverse primer, were designed by the inventors. Twenty-six amplifying primer pairs were designed to amplify DNA fragments of different sizes comprising portions of the promoter and the different exons and introns of the BRCA1 gene. Four additional primer pairs were designed to amplify control fragments, used as internal control (see Tables 1.1 and 1.2). As control region, the inventors selected 4 different regions that are usually not modified in humans due to important consequences, namely exon 5 of SMPD1, exon 3 of IL4, exon 8 of COL1A2 and exon 22 of COL1A1. Each amplifying primer was designed to comprise a tail sequence at the 5'-end that did not hybridize to the template DNA. The sequence of all the tails of the forward amplifying primers was identical among all them. The sequence of the tails of the reverse amplifying primers was the same, whereas the sequence of the tail of the forward amplifying primers was different from that of the reverse amplifying primers.

**Table 1.1. PCR primers pairs for BRCA1 gene and promoter and for the internal control**

| **Fragment** | **Primer** | **Primer sequence** | **SEQ ID NO** |
|---|---|---|---|
| E22 | E22F | AGGTCAGGATCAACGATGCAAAAGGACCCCATA | 1 |
| E15 | E15F | AGGTCAGGATCAACGAAATTCTTCTGGGGTCAG | 2 |
| E05 | C-SMPD1F | AGGTCAGGATCAACGTGGCCAGGTATGAGAACA | 3 |
| E04 | E04F | AGGTCAGGATCAACGCCATGAAAAGATAATCTC | 4 |
| E23 | E23F | AGGTCAGGATCAACGCAGAAGTCCTTTTCAGGCT | 5 |
| E2 | E02F | AGGTCAGGATCAACGTGTAAGGTCAATTCTGTT | 6 |
| E03 | C-IL4 | AGGTCAGGATCAACGTATCTGTGGCATTTGTCT | 7 |
| E10a | E10aF | AGGTCAGGATCAACGAGACAGACACTCGGTAGC | 8 |
| E21 | E21F | AGGTCAGGATCAACGAAGCACCACACAGCTGTA | 9 |
| E13 | E13F | AGGTCAGGATCAACGGGATTCTGGCTTATAGGG | 10 |
| E20 | E20F | AGGTCAGGATCAACGGGTTCTCCCAGGCTCTTA | 11 |
| E3 | E03F | AGGTCAGGATCAACGAGGTGTTTCCTGGGTTATG | 12 |
| E8 | E08F | AGGTCAGGATCAACGCAAACTGCACATACATCCC | 13 |
| E08 | C-COL1A2F | AGGTCAGGATCAACGAGGTTTCCAAGGACCTGCT | 14 |
| E01b | E01bF | AGGTCAGGATCAACGGTTAGCTAGGGGTGGGGTC | 15 |
| E10b | E10bF | AGGTCAGGATCAACGTGCAAGTTTGAAACAGAAC | 16 |
| Pr | Pr500F | AGGTCAGGATCAACGAGGCCTAGTTTCTGCTTTCA | 17 |
| E19 | E19F | AGGTCAGGATCAACGACCTTGGTGGTTTCTTCCA | 18 |
| E1 | E01F | AGGTCAGGATCAACGCAGTACCCCAGAGCATCAC | 19 |
| I5 | I05F | AGGTCAGGATCAACGACACCAACAATGTAAGTTG | 20 |
| E16 | E16F | AGGTCAGGATCAACGTTAGTTAAAGTGATGTGGT | 21 |
| E22 | C-COL1A1F | AGGTCAGGATCAACGGTTCACTGGCCTCCTCTCC | 22 |
| E7 | E07F | AGGTCAGGATCAACGTCACTTCCCAAAGCTGCC | 23 |
| E12 | nE12F | AGGTCAGGATCAACGCCTTCTAACAGCTACCCTT | 24 |
| E9 | E09F | AGGTCAGGATCAACGTCTTTTCAGTGCCTGTTAA | 25 |
| E17 | E17F | AGGTCAGGATCAACGTTAAAGACCTTTTGGTAAC | 26 |
| E14 | E14F | AGGTCAGGATCAACGAATCAAAGTGTTTGTTCCA | 27 |
| E13b | E13bF | AGGTCAGGATCAACGAAAGATATTCTAAATGTTT | 28 |
| I01 | I01F | AGGTCAGGATCAACGACCAAACCAACACCAATCA | 29 |
| 112 | I12F | AGGTCAGGATCAACGTCACAATAACATCAAGTCT | 30 |

The following letters when appear in the names of the primers indicates: E= exon; I= intron; Pr= promoter; C= primer for control fragment, F= forward; and R= reverse.

**Table 1.2**

| Fragment | Primer | Primer sequence | SEQ ID NO |
|---|---|---|---|
| E22 | E22R | CATCTTGCATGATCCAATGGCTTCCATGGTAAG | 31 |
| E15 | E15R | CATCTTGCATGATCCGTCAACAAAAGAATGTCC | 32 |
| E05 | grC-11 p15R | CATCTTGCATGATCCCCTCAAATTCATCCACAT | 33 |
| E04 | E04R | CATCTTGCATGATCCGGAAACTATTGCTTGTAA | 34 |
| E23 | nE23R | CATCTTGCATGATCCCTGGGAGCTCCTCTCACT | 35 |
| E2 | E02R | CATCTTGCATGATCCGTCCCATCTGGTAAGTCA | 36 |
| E03 | C-5q31F | CATCTTGCATGATCCCTCATGGTGGCTGTAGAA | 37 |
| E10a | E10aR | CATCTTGCATGATCCAAGAGCTTCCCTGCTTCC | 38 |
| E21 | nE21R | CATCTTGCATGATCCTAGGGTAGAGGGCCTGGGT | 39 |
| E13 | nE13R | CATCTTGCATGATCCTGAATTATCACTATCAGAAC | 40 |
| E20 | E20R | CATCTTGCATGATCCCCATTCCCCTGTCCCTCT | 41 |
| E3 | nE03R | CATCTTGCATGATCCTTGATCAAGGAACCTGTC | 42 |
| E8 | E08R | CATCTTGCATGATCCCAAAGAGAACCTTTGTCT | 43 |
| E08 | C1-R1 | CATCTTGCATGATCCATGGGAGACCCATCATTTC | 44 |
| E01b | E01bR | CATCTTGCATGATCCGGCTCTCTCATCCTGTCAC | 45 |
| E10b | E10bR | CATCTTGCATGATCCCTGCTTGTGAATTTTCTGA | 46 |
| Pr | Pr500R | CATCTTGCATGATCCTGGAGAGGAACATCCTAC | 47 |
| E19 | E19R | CATCTTGCATGATCCCTGGCCTGAATGCCTTAAAT | 48 |
| E1 | E01R | CATCTTGCATGATCCCGTGAGCTCGCTGAGACTTC | 49 |
| I5 | I05R | CATCTTGCATGATCCGGTCTCACACCTTATTTT | 50 |
| E16 | E16R | CATCTTGCATGATCCAGGACACGTGTAGAACGT | 51 |
| E22 | C2-R2 | CATCTTGCATGATCCTTTTGTGGCTCTTTGC | 52 |
| E7 | E07R | CATCTTGCATGATCCTGAGAACTCTGAGGACA | 53 |
| E12 | nE12R | CATCTTGCATGATCCTAAAATGTTGGAGCTAGG | 54 |
| E9 | E09R | CATCTTGCATGATCCTGGTCATTTGACAGTTCT | 55 |
| E17 | E17R | CATCTTGCATGATCCTTTGTGTGTGAACGGACA | 56 |
| E14 | E14R | CATCTTGCATGATCCTGGTACATGCACAGTTGC | 57 |
| E13b | E13bR | CATCTTGCATGATCCTTTCAGGCAATCCTC | 58 |
| I01 | I01R | CATCTTGCATGATCCAAGGGGAGGAGACAGGAT | 59 |
| I12 | nI12R | CATCTTGCATGATCCTGAGAAGCTTTCCATTAA | 60 |

The following letters when appear in the names of the primers indicates: E= exon; I= intron; Pr= promoter; C= primer for control fragment, F= forward; and R= reverse.

Additionally, a forward and a reverse labeling primer were designed. These primers consisted of the sequence of the tail of the forward and the reverse amplifying primers, respectively. The labeling primer containing the tail of the forward amplifying primers (namely, AGGTCAGGATCAACG sequence) was labeled with FAM at the 5'-end. The sequence of each reverse labeling primer was CATCTTGCATGATCC.

### PCR amplification conditions and amplicons analysis

Standard PCR kit was used for performing the PCR in a 200µl tube:

| | |
|---|---|
| 2x PCR reaction mix | 5 µL |
| Water | 0.75 µL |
| Primer mix (2 µM) | 2.25 µL |
| Template DNA (25ng/µL) | 2.0 µL |
| TOTAL VOLUME (per well | 10 µl |

The following optimized thermocycler conditions were used during the PCR:

| | | |
|---|---|---|
| 95 °C | 15' | |
| 95 °C | 30" | x 10 cycles |
| 60 °C | 30" | |
| 72 °C | 40" | |
| 95 °C | 30" | x 20 cycles |
| 65 °C | 30" | |
| 72 °C | 40" | |
| 72 °C | 15' | |
| 5-15 °C | ∞ | |

PCR products were loaded onto 3730 Genetic Analyzer (Applied Biosystem).

### Results

All previously described amplifying and labeling primers were used to amplify the DNA of two problem samples, namely two samples obtained from IBC (inherited breast cancer) affected subjects with large rearrangements, and three control samples from healthy subjects, used as controls. Two replicates (i.e. replicates 1 and 2) were obtained for each sample of the IBC affected subjects, and for the controls, in order to check inter-experimental variation. Fig. 5 shows in each panel (panels A and B) the results obtained for the two replicates. As shown therein, 30 peaks were obtained corresponding to the 30 amplified products using the 30 primer pairs of Table 1. As depicted in Fig. 5, "*" indicates those peaks with reduced intensity compared to the intensity of the same peak observed in the healthy samples. After normalization of peak intensities by using the peak intensity of the amplification products obtained with the control primers, the standard deviation obtained was less than 5% for all the amplified fragments (see Fig. 6), except for the peaks under the stars, which showed an intensity between approximately 35-60% of the intensity of the same peaks in the control samples. This intensity reduction indicated a deletion of the corresponding amplified fragment. Accordingly, as it can be seen in Fig. 7 the sample on panel A had a deletion of exons 3 and 4. And the sample on panel B had a deletion of the promoter up to exon 12.

The results obtained demonstrated the ability of the method of the present invention to detect large rearrangements along the entire length of the promoter and the BRCA1 human gene. Over 20 additional samples from IBC affected subjects were tested and in all cases the results obtained following the described method of the present invention allowed the detection of large rearrangements.

### Example 2. SNPs Genotyping the promoter of the human lactase gene

### PCR primers

We have designed primers for the detection of 4 SNPs in the lactase promoter gene, namely rs41525747, rs4988235, rs41380347 and rs182549 (see Table 2). In particular, two forward amplifying PCR primers for each SNP were included. Both primers were ASO primers for genotyping the two alleles of each SNP, each primer with a tail at the 5'-end. The sequences of the tails did not hybridize to the template DNA, and were common to all the forward amplifying primers. Two reverse primers, each of them with a tail at the 3'-end, were added, wherein the sequence of this tail was the same for both reverse primers, and different from the sequences of the tails of the forward amplifying primers. For the genotyping of SNPs rs41525747, rs4988235 and rs41380347 the same reverse amplifying primer was used, namely L-13900-3 (REV). Spacer sequences between the tail and the ASO primers were introduced when needed (see Table 2) so that the resulting amplification products of each allele of the 4 tested SNPs gave different sizes. A forward and reverse labeling primer were also designed and added to the PCR one-step reaction. The forward and the reverse labeling primers comprised the sequence of the tail of the forward and the reverse amplifying primer, respectively. Only one of the pair of labeling primers was labeled with fluorescein.

**Table 2. Primers for SNPs genotyping in the promoter of the human lactamase gene**

| Primer name | Sequence (5'→3') | SEQ ID NO |
|---|---|---|
| L-rs41525747 G-5 | AGGTCAGGATCAACGCAATACAGATAAGATAATGTAGCCCG | 61 |
| L-rs41525747 C-5 | AGGTCAGGATCAACGACTCCAATACAGATAAGATAATGTAGCCCC | 62 |
| L-rs4988235 T-5 | AGGTCAGGATCAACGCTCTAGTGGCAATACAGATAAGATAATGTAGT | 63 |
| L-rs4988235 C-5 | AGGTCAGGATCAACGACGTGTGTTATGGCAATACAGATAAGATAATGTAGC | 64 |
| L-rs41380347 G-5 | AGGTCAGGATCAACGTTGATGGAGTCACGCTGGCAATACAGATAAGATAAG | 65 |
| L-rs41380347 T-5 | AGGTCAGGATCAACGTACTCGTAGGCCTCTGCGCTGGCAATACAGATAA- GATAAT | 66 |
| L-rs 182549 A-5 | AGGTCAGGATCAACGAGCATTCTCAGCTGGGCA | 67 |
| L-rs 182549 G-5 | AGGTCAGGATCAACGTATAGAGCATTCTCAGCTGGGCG | 68 |
| L-13900-3 (REV) | CATCTTGCATGATCCAGGGCTGCTTTGGTTGAAG | 69 |
| L-rs182549 (REV) | CATCTTGCATGATCCTGGCACAATCTTGGCTCA | 70 |

Underlined primer sequence corresponds to the 5'-end tails. Primer sequence in italics corresponds to the spacers. REV stands for reverse primer

### PCR amplification conditions and amplicons analysis

Standard PCR kit was used for performing the PCR in a 200µl tube:

| | |
|---|---|
| 2x PCR reaction mix | 5 µL |
| Water | 0.75 µL |
| Primer mix (2 µM) | 2.25 µL |
| Template DNA (25ng/µL) | 2.0 µL |
| TOTAL VOLUME (per well) | 10 µl |

The following optimized thermocycler conditions were used during the PCR:

| | | |
|---|---|---|
| 95 °C | 15' | |
| 95 °C | 30" | x 10 cycles |
| 60 °C | 30" | |
| 72 °C | 40" | |
| 95 °C | 30" | x 20 cycles |
| 65 °C | 30" | |
| 72 °C | 40" | |
| 72 °C | 15' | |
| 5-15 °C | ∞ | |

PCR products were loaded onto 3730 Genetic Analyzer (Applied Biosystem).

After the PCR reaction, the products were loaded onto a Capillary Genetic Analyzer, namely a Capillary DNA Sequencer, for fragment analysis sizing and quantification based on the detection of the fluorescein.

### Results

Over 20 samples were analyzed with the above primers sets. The sizes of the obtained amplicons are disclosed in Table 3 below.

**Table 3**

| SNP | Fragment size (bp) |
|---|---|
| rs41525747G | 240 |
| rs41525747C | 244 |
| rs4988235T | 249 |
| rs4988235C | 253 |
| rs41380347G | 258 |
| rs41380347T | 262 |
| rs182549A | 293 |
| rs182549G | 298 |

Fig. 8 shows the peaks corresponding to the 4 genotyped SNPs obtained in the 3730 Genetic Analyzer (Applied Biosystem) for 1 out of the 20 analyzed samples. In particular, peak 1 corresponds to the homozygous genotype CC of the rs41525747 SNP; peaks 2 and 3 correspond to the heterozygous genotype CT of the rs4988235 SNP; peak 4 corresponds to the homozygous genotype TT of the rs41380347 SNP; and peaks 5 and 6 corresponds to the heterozygous genotype AG of the rs182549 SNP. The obtained genotypes applying the described method of the present invention fully agreed with those determined by Next Generation System.

### Example 3: Determination of the HLA DQA1*01 and HLA DQA1*03 haplotypes

### PCR primers

PCR primers pairs for the detection of haplotypes DQA1*01 and *03 are shown in Table 4. PCR amplifying primers were designed to include in the forward and reverse amplifying primers several polymorphisms, so that the haplotype could be determined. A forward and a reverse amplifying primer were designed with a sequence comprising the nucleotides in the polymorphic positions corresponding to haplotype HLA DQA1*01, and a tail at the 5'-end. The sequences of the tails of the forward and reverse amplifying primers were different from each other. A second pair of forward and reverse amplifying primers were also designed for haplotype HLA DQA1*03. The tail sequences of the two forward amplifying primers were identical between them. Similarly, the tail sequences of the two reverse amplifying primers were identical between them. The sequences of both tails did not hybridize to the target DNA. Additionally, spacer sequences at the 3'-end of the tails were introduced in some of the amplifying primers as shown in Table 4 so that the resulting amplification products of the two sets of primers gave different sizes for each haplotype. A forward and a reverse labeling primer comprising the sequence of the tail of the forward and reverse amplifying primer, respectively, were also included in the PCR reaction. Only one of the two labeling primers was labeled with FAM. The expected fragment sizes were 126 bp and 134 bp for DQA1*01 and DQA1*03, respectively.

**Table 4. Primers for the determination of the HLA DQA1*01 and HLA DQA1*03 haplotypes**

| Primer name | Sequence (5'→3') | SEQ ID NO |
|---|---|---|
| 5-DQA1*01f | ACACCCTGCAGCTGTTCTTCGT**G**GCCT**GA**GTTC**A**GCA**A** | 71 |
| 3-DQA1*01r | GTCGGAACTCTGCCTCTTCTGA**T**GTTCAAGTT*G*TGTTTTGC | 72 |
| 5-DQA1*03f | ACACCCTGCAGCTGTTCTTC*AG*T**T**GCCT*CT*GTTCCGCAG | 73 |
| 3-DQA1*03r | GTCGGAACTCTGCCTCTTCT*CACG*ATGTTCAAGTT**A**TGTTTT**A**C | 74 |

Underlined primer sequence corresponds to the 5'-end tails. Primer sequence in italics corresponds to the spacers. Primer sequence in bold corresponds to the polymorphic positions

### PCR amplification conditions and amplicons analysis

Standard PCR kit was used for performing the PCR in a 200µl tube:

| | |
|---|---|
| 2x PCR reaction mix | 7.5 µL |
| Water | 4.5 µL |
| Primer mix (2 µM) | 1.0 µL |
| Template DNA (25ng/µL) | 2.0 µL |
| TOTAL VOLUME (per well) | 15 µl |

The following optimized thermocycler conditions were used during the PCR:

| | | |
|---|---|---|
| 95 °C | 15' | |
| 95 °C | 30" | |
| 60 °C | 30" | x 10 cycles |
| 72 °C | 40" | |
| 95 °C | 30" | |
| 65 °C | 30" | x 20 cycles |
| 72 °C | 40" | |
| 72 °C | 15' | |
| 5-15 °C | ∞ | |

After the PCR reaction, the amplified products were loaded into a Genetic Analyzer (Capillary DNA Sequencer) for fragment analysis and quantification based on the detection of the label.

### Results

Over 25 samples were analyzed with the above primers sets. Fig. 9 shows the peaks of 126 bp and 134 bp corresponding to ther DQA1*01 and DQA1*03 haplotypes obtained in the 3730 Genetic Analyzer (Applied Biosystem) for one of the 25 analyzed samples. The obtained haplotypes applying the method of the present invention fully agreed with the genotypes determined by sequencing.

### Example 4: Amplification, barcoding and final library preparation for NGS in one step

### PCR primers

A PCR reaction for amplification of exons 2 and 3 of the human KRAS gene from a test sample was performed in order to detect mutations using a NGS.

PCR amplifying primers were designed as in previous examples, but considering the specific sequences needed for sequencing with the GS454 Junior System (Roche). PCR primer pairs are shown in Table 5 below.

**Table 5**

| Primer name | Sequence (5'→3') | SEQ ID NO |
|---|---|---|
| KRAS-E2-f | AGGTCAGGATCAACGCTCAAGTTTTATTATAAGGCCTGC | 75 |
| KRAS-E2-r | CATCTTGCATGATCCAACCTTCGTACTCATGAAAATGGTCA | 76 |
| KRAS-E3-f | AGGTCAGGATCAACGCTCAAGGTGTTTCTCCCTTCTCAG | 77 |
| KRAS-E3-r | CATCTTGCATGATCCAACCTTCTTTATGGCAAATACACAA | 78 |
| A-K-1-D5f | cgtatcgcctccctcgcgccaTCAGACGAGTGCGTAGGTCAGGATCAACGC | 79 |
| A-K-2-D5f | cgtatcgcctccctcgcgccaTCAGACGCTCGACAAGGTCAGGATCAACGC | 80 |
| A-K-3-D5f | cgtatcgcctccctcgcgccaTCAGAGACGCACTCAGGTCAGGATCAACGC | 81 |
| B-K-1-D3r | **ctatgcgccttgccagcccgc**TCAGACGAGTGCGTCATCTTGCATGATCCA | 82 |
| B-K-2-D3r | **ctatgcgccttgccagcccgc**TCAGACGCTCGACACATCTTGCATGATCCA | 83 |
| B-K-3-D3r | **ctatgcgccttgccagcccgc**TCAGAGACGCACTCCATCTTGCATGATCCA | 84 |

Underlined primer sequence corresponds to the tails. Primer sequence in lower case letter corresponds to sequence A. Primer sequence in bold and in lower case letter corresponds to sequence B described in GS Junior Protocols.

### PCR amplification conditions and amplicons analysis

Standard PCR kit was used for performing the PCR in a 200µl tube:

| | |
|---|---|
| 2x PCR reaction mix | 7.5 µL |
| Water | 4.5 µL |
| Primer mix (2 µM) | 1.0 µL |
| Template DNA (25ng/µL) | 2.0 µL |
| TOTAL VOLUME (per well) | 15 µl |

The following optimized thermocycler conditions were used during the PCR:

| | | |
|---|---|---|
| 95 °C | 10' | |
| 95 °C | 30" | |
| 60 °C | 30" | x 10 cycles |
| 72 °C | 40" | |
| 95 °C | 30" | |
| 65 °C | 30" | x 20 cycles |
| 72 °C | 40" | |
| 72 °C | 15' | |
| 5-15 °C | ∞ | |

PCR products were loaded onto Qiaxcel System (Qiagen) in order to calculate the size of the amplified products and then proceed with the sequencing in GS454 Junior System (Roche), a New Geeration Sequencing System. A control reaction for including barcodes was performed by standard procedure, based on two PCR steps, one for the amplification and the second step for barcoding and inclusion of the rest of sequencing sequences.

### Results

An amplicon of 261 bp was obtained in the first PCR reaction of a two-steps protocol for including the tails corresponding to exon 2 of the human KRAS gene (A). A second amplicon of 332 bp corresponding to exon 2 was obtained in the second PCR reaction after the first PCR reaction, which showed the increase in the product size due to inclusion of barcoding primers (B). A single amplicon of 332 bp corresponding to exon 2, obtained in the method of the invention, wherein the size of the peak agreed with the size expected after inclusion of barcoding primers (C). Two amplicons of 209 and 261 bp, corresponding to exon 3 and 2, respectively, of the human KRAS gene, were obtained in the first PCR reaction of a two-steps protocol for including the tails (D). Two amplicons of 280 and 332 bp corresponding to exon 3 and 2, respectively, were obtained in the second PCR reaction under standard protocol, which showed the increase in the product size due to inclusion of barcoding primers (E). Two amplicons of 280 and 332 bp corresponding to exon 3 and 2, respectively, of the human KRAS gene, were obtained following the one-step method of the invention, wherein the size of the peak agreed with the size expected after inclusion of barcoding primers (F).

## Claims

1. A one-step PCR-based in vitro method for the generation of labeled amplicons from at least a nucleic acid target region in a sample, wherein at least two pairs of PCR primers are used to obtain the labeled amplicons:
- a first pair of PCR amplifying primers comprising a reverse and a forward primer, wherein each PCR amplifying primer comprises at least two regions, a first region or tail, located at the 5'-end, which is not complementary to the at least nucleic acid target region, and a second region, located at the 3'-end, which is sufficiently complementary to the at least nucleic acid target region to allow the amplification of the at least nucleic acid target region to obtain amplicons; and wherein the sequence of the tail of the primers is different from each other;
- a second pair of PCR labeling primers, wherein one of the labeling primers has a sequence which is sufficiently identical to the tail of one of the amplifying primers, and the other labeling primer has a sequence which is sufficiently identical to the tail of the other amplifying primer, to allow amplification of the amplicons obtained using the first pair of PCR amplifying primes, and wherein at least one of the primers of the second pair of PCR labeling primers is labeled.

2. The method according to the claim 1, wherein the PCR-based in vitro method occurs in a single reaction, which comprises one or more experimental conditions of amplification cycles.

3. The method according to any one of claims 1 or 2, wherein the sample is selected from the group consisting of human, animal, plant, fungal, bacterial, synthetic nucleic acids and viral sample.

4. The method according to any one of claims 1 to 3, wherein at least one of the primers of the second pair of PCR labeling primers is labeled with a label selected from the group consisting of: fluorescent label, chemical label, radioactive label, and nucleotide sequence label located at the 5'-end region.

5. The method according to claim 4 wherein each primer of the second pair of PCR labeling primers has a nucleotide sequence label located at the 5'-end.

6. The method according to claim 4, wherein only one of the second pair of PCR labeling primers is labeled.

7. The method according to any one claims 1 to 6, wherein at least one primer of the first pair of PCR amplifying primers comprises a nucleotide spacer located at the 3'-end of the tail.

8. The method according to any one of claims 1 to 7, wherein the labeled amplicons are sized, so that if the size of the labeled amplicons is increased when compared to a size reference value, then it is indicative of a small or a large insertion, depending on the increase, and if the size is decreased when compared to a size reference value, then it is indicative of a small or a large deletion, depending on the decrease.

9. The method according to any one of claims 1 to 7, wherein the labeled amplicons are sized and quantified, so that if the size of the labeled amplicons is that of a size reference value and if the quantity of the labeled amplicons is increased when compared to a quantity reference value, then it is indicative of an amplified copy number variation (CNV), and if the size of the labeled amplicons is that of a size reference value and if the quantity of the labeled amplicons is decreased when compared to a quantity of reference value then it is indicative of a reduced CNV.

10. The method according to any one of claims 1 to 7 for haplotyping, wherein at least one of the primers of the first pair of PCR amplifying primers contains at different nucleotide positions two or more alleles to determine a haplotype.

11. The method according to any one of claims 1 to 7 for allele genotyping, wherein at least one of the primers of the first pair of PCR amplifying primers is an ASO primer.

12. The method according to any one of claims 1 to 11, wherein the sequence of the labeled amplicons is determined.

13. The method according to the claim 12, wherein the at least nucleic acid target region is a region of a gene selected from the group consisting of the ABCA1, ABCB11, ABCG5, ABCG8, AKT, ALK, ANK2, APC, APOA1, APOB, APOC2, APOE, APP, ARH, ASXL1, ATM, ATP8B1, BIRC3, BRCA1, BRCA2, CBS, CEBPA, CETP, CFTR, CKIT, CLCNKB, CLDN16, CLDN19, COL1A1, COL1A2, COL3A1, CYP21A2, DAX1, DMD, DMGDH, DNMT3A, EFGFR, EGR2, EPCAM, ERBB2, ERBB3, ERS1, FBN1, FBXW7, FGFR1, FGFR2, FGFR3, FGFR4, FHF6, FLCN, FLT3-ITD, FM01, FM03, FRAF, GCK, GNA11 , GNAQ, HNF1A, HNF1B, HNF4A, HRAS, IDH1, IDH2, KCNE1, KCNE2, KCNH2, KCNJ2, KCNQ1, KEAP1, LCAT, LDLR, LPL, MEFV, MEK1, MEN1, MEN2, MLH1, MSH2, MTHFR, MTP, MYD88, NF1, NF1, NF2, NFKBIE, NOTCH1, NRAS, NRAS, PAX8, PCSK9, PDGFRA, PIK3CA, PKP2, POT1, PRKAR1A, PRSS1, PSEN1, PTEN, RET, RET, RICTOR, ROS1, RUNX1, RUNX1, SCN5A, SDHB, SDHC, SDHD, SERPINA1, SF3B1, SLC12A3, SLC22A1, SLC34A2, SMAD4, SMN1, SMO, SPINK1, STK11, TET2, TGFBR1, TGFBR2, TP53, and XPO1 genes

14. A kit for one-step PCR-based in vitro method for the generation of labeled amplicons from at least a nucleic acid target region in a sample, comprising at least two pairs of PCR primers:
- a first pair of PCR amplifying primers comprising a reverse and a forward primer, wherein each PCR amplifying primer comprises at least two regions, a first region or tail, located at the 5'-end, which is not complementary to the at least nucleic acid target region, and a second region, located at the 3'-end, which is sufficiently complementary to the at least nucleic acid target region to allow the amplification of the at least nucleic acid target region to obtain amplicons; and wherein the sequence of the tail of the primers is different from each other;
- a second pair of PCR labeling primers, wherein one of the labeling primers has a sequence which is sufficiently identical to the tail of one of the amplifying primers, and the other labeling primer has a sequence which is sufficiently identical to the tail of the other amplifying primer, to allow amplification of the amplicons obtained using the first pair of PCR amplifying primes, and wherein at least one of the primers of the second pair of PCR labeling primers is labeled.

15. Use of the kit according to claim 14 in the diagnosis of a disease involving one or more large rearrangements, small mutations, genetic polymorphisms, CNVs and combinations thereof.

16. Use of the kit according to any one of claims 14 to 15, wherein the at least nucleic acid target region is a region of a gene selected from the group consisting of ABCA1, ABCB11, ABCG5, ABCG8, AKT, ALK, ANK2, APC, APOA1, APOB, APOC2, APOE, APP, ARH, ASXL1, ATM, ATP8B1, BIRC3, BRCA1, BRCA2, CBS, CEBPA, CETP, CFTR, CKIT, CLCNKB, CLDN16, CLDN19, COL1A1, COL1A2, COL3A1, CYP21A2, DAX1, DMD, DMGDH, DNMT3A, EFGFR, EGR2, EPCAM, ERBB2, ERBB3, ERS1, FBN1, FBXW7, FGFR1, FGFR2, FGFR3, FGFR4, FHF6, FLCN, FLT3-ITD, FM01, FM03, FRAF, GCK, GNA11 , GNAQ, HNF1A, HNF1B, HNF4A, HRAS, IDH1, IDH2, KCNE1, KCNE2, KCNH2, KCNJ2, KCNQ1, KEAP1, LCAT, LDLR, LPL, MEFV, MEK1, MEN1, MEN2, MLH1, MSH2, MTHFR, MTP, MYD88, NF1, NF1, NF2, NFKBIE, NOTCH1, NRAS, NRAS, PAX8, PCSK9, PDGFRA, PIK3CA, PKP2, POT1, PRKAR1A, PRSS1, PSEN1, PTEN, RET, RET, RICTOR, ROS1, RUNX1, RUNX1, SCN5A, SDHB, SDHC, SDHD, SERPINA1, SF3B1, SLC12A3, SLC22A1, SLC34A2, SMAD4, SMN1, SMO, SPINK1, STK11, TET2, TGFBR1, TGFBR2, TP53, and XPO1 genes.

17. Use of the kit according to any one of claims 14 to 16, wherein the disease is selected from the group consisting of familial hypercholesterolemia, breast cancer and ovarian cancer.

18. Use of the kit according to claim 14 to generate a group of labeled amplicons from at least a nucleic acid target region to generate NGS libraries, for DNA detection, DNA characterization, DNA sequencing and/or DNA hybridization.


SEQUENCE LISTING

<110> SEQUENCING MULTIPLEX S.L.
FUNDACIÓN PARA LA INVESTIGACIÓN DEL HOSPITAL CLÍNICO
UNIVERSITARIO DE LA COMUNIDAD VALENCIANA, INSTITUTO DE
INVESTIGACIÓN SANITARIA CLÍNICO DE VALENCIA (INCLIVA)

<120> EASY ONE-STEP AMPLIFICATION AND LABELING (EOSAL)

<130> PD10159EP00

<160> 84

<170> PatentIn version 3.5

<210> 1
<211> 33
<212> DNA
<213> Artificial Sequence

<220>
<223> Forward primer for human BRCA1 gene

<400> 1
aggtcaggat caacgatgca aaaggacccc ata 33

<210> 2
<211> 33
<212> DNA
<213> Artificial Sequence

<220>
<223> Forward primer for human BRCA1 gene

<400> 2
aggtcaggat caacgaaatt cttctggggt cag 33

<210> 3
<211> 33
<212> DNA
<213> Artificial Sequence

<220>
<223> Forward primer for human BRCA1 gene

<400> 3
aggtcaggat caacgtggcc aggtatgaga aca 33

<210> 4
<211> 33
<212> DNA
<213> Artificial Sequence

<220>
<223> Forward primer for human BRCA1 gene

<400> 4
aggtcaggat caacgccatg aaaagataat ctc 33

<210>  5
<211>  34
<212>  DNA
<213>  Artificial Sequence

<220>
<223>  Forward primer for human BRCA1 gene

<400>  5
aggtcaggat caacgcagaa gtccttttca ggct                                    34


<210>  6
<211>  33
<212>  DNA
<213>  Artificial Sequence

<220>
<223>  Forward primer for human BRCA1 gene

<400>  6
aggtcaggat caacgtgtaa ggtcaattct gtt                                     33


<210>  7
<211>  33
<212>  DNA
<213>  Artificial Sequence

<220>
<223>  Forward primer for human BRCA1 gene

<400>  7
aggtcaggat caacgtatct gtggcatttg tct                                     33


<210>  8
<211>  33
<212>  DNA
<213>  Artificial Sequence

<220>
<223>  Forward primer for human BRCA1 gene

<400>  8
aggtcaggat caacgagaca gacactcggt agc                                     33


<210>  9
<211>  33
<212>  DNA
<213>  Artificial Sequence

<220>
<223>  Forward primer for human BRCA1 gene

<400>  9
aggtcaggat caacgaagca ccacacagct gta                                     33


<210>  10
<211>  33
<212>  DNA

<213>  Artificial Sequence

<220>
<223>  Forward primer for human BRCA1 gene

<400>  10
aggtcaggat caacgggatt ctggcttata ggg                                    33


<210>  11
<211>  33
<212>  DNA
<213>  Artificial Sequence

<220>
<223>  Forward primer for human BRCA1 gene

<400>  11
aggtcaggat caacgggttc tcccaggctc tta                                    33


<210>  12
<211>  34
<212>  DNA
<213>  Artificial Sequence

<220>
<223>  Forward primer for human BRCA1 gene

<400>  12
aggtcaggat caacgaggtg tttcctgggt tatg                                   34


<210>  13
<211>  34
<212>  DNA
<213>  Artificial Sequence

<220>
<223>  Forward primer for human BRCA1 gene

<400>  13
aggtcaggat caacgcaaac tgcacataca tccc                                   34


<210>  14
<211>  34
<212>  DNA
<213>  Artificial Sequence

<220>
<223>  Forward primer for human BRCA1 gene

<400>  14
aggtcaggat caacgaggtt tccaaggacc tgct                                   34


<210>  15
<211>  34
<212>  DNA
<213>  Artificial Sequence

<220>

<223>  Forward primer for human BRCA1 gene

<400>  15
aggtcaggat caacggttag ctaggggtgg ggtc                                34


<210>  16
<211>  34
<212>  DNA
<213>  Artificial Sequence

<220>
<223>  Forward primer for human BRCA1 gene

<400>  16
aggtcaggat caacgtgcaa gtttgaaaca gaac                                34


<210>  17
<211>  35
<212>  DNA
<213>  Artificial Sequence

<220>
<223>  Forward primer for human BRCA1 gene

<400>  17
aggtcaggat caacgaggcc tagtttctgc tttca                               35


<210>  18
<211>  34
<212>  DNA
<213>  Artificial Sequence

<220>
<223>  Forward primer for human BRCA1 gene

<400>  18
aggtcaggat caacgacctt ggtggtttct tcca                                34


<210>  19
<211>  34
<212>  DNA
<213>  Artificial Sequence

<220>
<223>  Forward primer for human BRCA1 gene

<400>  19
aggtcaggat caacgcagta ccccagagca tcac                                34


<210>  20
<211>  34
<212>  DNA
<213>  Artificial Sequence

<220>
<223>  Forward primer for human BRCA1 gene

<400>  20

aggtcaggat caacgacacc aacaatgtaa gttg                                    34

<210>  21
<211>  34
<212>  DNA
<213>  Artificial Sequence

<220>
<223>  Forward primer for human BRCA1 gene

<400>  21
aggtcaggat caacgttagt taaagtgatg tggt                                    34

<210>  22
<211>  34
<212>  DNA
<213>  Artificial Sequence

<220>
<223>  Forward primer for human BRCA1 gene

<400>  22
aggtcaggat caacggttca ctggcctcct ctcc                                    34

<210>  23
<211>  33
<212>  DNA
<213>  Artificial Sequence

<220>
<223>  Forward primer for human BRCA1 gene

<400>  23
aggtcaggat caacgtcact tcccaaagct gcc                                     33

<210>  24
<211>  34
<212>  DNA
<213>  Artificial Sequence

<220>
<223>  Forward primer for human BRCA1 gene

<400>  24
aggtcaggat caacgccttc taacagctac cctt                                    34

<210>  25
<211>  34
<212>  DNA
<213>  Artificial Sequence

<220>
<223>  Forward primer for human BRCA1 gene

<400>  25
aggtcaggat caacgtcttt tcagtgcctg ttaa                                    34

<210>  26
<211>  34
<212>  DNA
<213>  Artificial Sequence

<220>
<223>  Forward primer for human BRCA1 gene

<400>  26
aggtcaggat caacgttaaa gaccttttgg taac                                    34


<210>  27
<211>  34
<212>  DNA
<213>  Artificial Sequence

<220>
<223>  Forward primer for human BRCA1 gene

<400>  27
aggtcaggat caacgaatca aagtgtttgt tcca                                    34


<210>  28
<211>  34
<212>  DNA
<213>  Artificial Sequence

<220>
<223>  Forward primer for human BRCA1 gene

<400>  28
aggtcaggat caacgaaaga tattctaaat gttt                                    34


<210>  29
<211>  34
<212>  DNA
<213>  Artificial Sequence

<220>
<223>  Forward primer for human BRCA1 gene

<400>  29
aggtcaggat caacgaccaa accaacacca atca                                    34


<210>  30
<211>  34
<212>  DNA
<213>  Artificial Sequence

<220>
<223>  Forward primer for human BRCA1 gene

<400>  30
aggtcaggat caacgtcaca ataacatcaa gtct                                    34


<210>  31
<211>  33
<212>  DNA

<213>  Artificial Sequence

<220>
<223>  Reverse primer for human BRCA1 gene

<400>  31
catcttgcat gatccaatgg cttccatggt aag                                    33


<210>  32
<211>  33
<212>  DNA
<213>  Artificial Sequence

<220>
<223>  Reverse primer for human BRCA1 gene

<400>  32
catcttgcat gatccgtcaa caaaagaatg tcc                                    33


<210>  33
<211>  33
<212>  DNA
<213>  Artificial Sequence

<220>
<223>  Reverse primer for human BRCA1 gene

<400>  33
catcttgcat gatcccctca aattcatcca cat                                    33


<210>  34
<211>  33
<212>  DNA
<213>  Artificial Sequence

<220>
<223>  Reverse primer for human BRCA1 gene

<400>  34
catcttgcat gatccggaaa ctattgcttg taa                                    33


<210>  35
<211>  33
<212>  DNA
<213>  Artificial Sequence

<220>
<223>  Reverse primer for human BRCA1 gene

<400>  35
catcttgcat gatccctggg agctcctctc act                                    33


<210>  36
<211>  33
<212>  DNA
<213>  Artificial Sequence

<220>

<223>  Reverse primer for human BRCA1 gene

<400>  36
catcttgcat gatccgtccc atctggtaag tca                                    33


<210>  37
<211>  33
<212>  DNA
<213>  Artificial Sequence

<220>
<223>  Reverse primer for human BRCA1 gene

<400>  37
catcttgcat gatccctcat ggtggctgta gaa                                    33


<210>  38
<211>  33
<212>  DNA
<213>  Artificial Sequence

<220>
<223>  Reverse primer for human BRCA1 gene

<400>  38
catcttgcat gatccaagag cttccctgct tcc                                    33


<210>  39
<211>  34
<212>  DNA
<213>  Artificial Sequence

<220>
<223>  Reverse primer for human BRCA1 gene

<400>  39
catcttgcat gatcctaggg tagagggcct gggt                                   34


<210>  40
<211>  35
<212>  DNA
<213>  Artificial Sequence

<220>
<223>  Reverse primer for human BRCA1 gene

<400>  40
catcttgcat gatcctgaat tatcactatc agaac                                  35


<210>  41
<211>  33
<212>  DNA
<213>  Artificial Sequence

<220>
<223>  Reverse primer for human BRCA1 gene

<400>  41

catcttgcat gatccccatt cccctgtccc tct                                    33

<210>  42
<211>  33
<212>  DNA
<213>  Artificial Sequence

<220>
<223>  Reverse primer for human BRCA1 gene

<400>  42
catcttgcat gatccttgat caaggaacct gtc                                    33

<210>  43
<211>  33
<212>  DNA
<213>  Artificial Sequence

<220>
<223>  Reverse primer for human BRCA1 gene

<400>  43
catcttgcat gatcccaaag agaacctttg tct                                    33

<210>  44
<211>  34
<212>  DNA
<213>  Artificial Sequence

<220>
<223>  Reverse primer for human BRCA1 gene

<400>  44
catcttgcat gatccatggg agacccatca tttc                                   34

<210>  45
<211>  34
<212>  DNA
<213>  Artificial Sequence

<220>
<223>  Reverse primer for human BRCA1 gene

<400>  45
catcttgcat gatccggctc tctcatcctg tcac                                   34

<210>  46
<211>  34
<212>  DNA
<213>  Artificial Sequence

<220>
<223>  Reverse primer for human BRCA1 gene

<400>  46
catcttgcat gatccctgct tgtgaatttt ctga                                   34

<210>  47
<211>  33
<212>  DNA
<213>  Artificial Sequence

<220>
<223>  Reverse primer for human BRCA1 gene

<400>  47
catcttgcat gatcctggag aggaacatcc tac                                    33


<210>  48
<211>  35
<212>  DNA
<213>  Artificial Sequence

<220>
<223>  Reverse primer for human BRCA1 gene

<400>  48
catcttgcat gatccctggc ctgaatgcct taaat                                  35


<210>  49
<211>  35
<212>  DNA
<213>  Artificial Sequence

<220>
<223>  Reverse primer for human BRCA1 gene

<400>  49
catcttgcat gatcccgtga gctcgctgag acttc                                  35


<210>  50
<211>  33
<212>  DNA
<213>  Artificial Sequence

<220>
<223>  Reverse primer for human BRCA1 gene

<400>  50
catcttgcat gatccggtct cacaccttat ttt                                    33


<210>  51
<211>  33
<212>  DNA
<213>  Artificial Sequence

<220>
<223>  Reverse primer for human BRCA1 gene

<400>  51
catcttgcat gatccaggac acgtgtagaa cgt                                    33


<210>  52
<211>  31
<212>  DNA

<213>  Artificial Sequence

<220>
<223>  Reverse primer for human BRCA1 gene

<400>  52
catcttgcat gatccttttg tggctctttg c                                    31


<210>  53
<211>  32
<212>  DNA
<213>  Artificial Sequence

<220>
<223>  Reverse primer for human BRCA1 gene

<400>  53
catcttgcat gatcctgaga actctgagga ca                                  32


<210>  54
<211>  33
<212>  DNA
<213>  Artificial Sequence

<220>
<223>  Reverse primer for human BRCA1 gene

<400>  54
catcttgcat gatcctaaaa tgttggagct agg                                 33


<210>  55
<211>  33
<212>  DNA
<213>  Artificial Sequence

<220>
<223>  Reverse primer for human BRCA1 gene

<400>  55
catcttgcat gatcctggtc atttgacagt tct                                 33


<210>  56
<211>  33
<212>  DNA
<213>  Artificial Sequence

<220>
<223>  Reverse primer for human BRCA1 gene

<400>  56
catcttgcat gatcctttgt gtgtgaacgg aca                                 33


<210>  57
<211>  33
<212>  DNA
<213>  Artificial Sequence

<220>

<223>  Reverse primer for human BRCA1 gene

<400>  57
catcttgcat gatcctggta catgcacagt tgc                                    33


<210>  58
<211>  30
<212>  DNA
<213>  Artificial Sequence

<220>
<223>  Reverse primer for human BRCA1 gene

<400>  58
catcttgcat gatcctttca ggcaatcctc                                        30


<210>  59
<211>  33
<212>  DNA
<213>  Artificial Sequence

<220>
<223>  Reverse primer for human BRCA1 gene

<400>  59
catcttgcat gatccaaggg gaggagacag gat                                    33


<210>  60
<211>  33
<212>  DNA
<213>  Artificial Sequence

<220>
<223>  Reverse primer for human BRCA1 gene

<400>  60
catcttgcat gatcctgaga agctttccat taa                                    33


<210>  61
<211>  41
<212>  DNA
<213>  Artificial Sequence

<220>
<223>  Forward primer for the promoter of the human lactamase gene

<400>  61
aggtcaggat caacgcaata cagataagat aatgtagccc g                           41


<210>  62
<211>  45
<212>  DNA
<213>  Artificial Sequence

<220>
<223>  Forward primer for the promoter of the human lactamase gene

<400>  62

aggtcaggat caacgactcc aatacagata agataatgta gcccc 45

<210> 63
<211> 47
<212> DNA
<213> Artificial Sequence

<220>
<223> Forward primer for the promoter of the human lactamase gene

<400> 63
aggtcaggat caacgctcta gtggcaatac agataagata atgtagt 47

<210> 64
<211> 51
<212> DNA
<213> Artificial Sequence

<220>
<223> Forward primer for the promoter of the human lactamase gene

<400> 64
aggtcaggat caacgacgtg tgttatggca atacagataa gataatgtag c 51

<210> 65
<211> 51
<212> DNA
<213> Artificial Sequence

<220>
<223> Forward primer for the promoter of the human lactamase gene

<400> 65
aggtcaggat caacgttgat ggagtcacgc tggcaataca gataagataa g 51

<210> 66
<211> 55
<212> DNA
<213> Artificial Sequence

<220>
<223> Forward primer for the promoter of the human lactamase gene

<400> 66
aggtcaggat caacgtactc gtaggcctct gcgctggcaa tacagataag ataat 55

<210> 67
<211> 33
<212> DNA
<213> Artificial Sequence

<220>
<223> Forward primer for the promoter of the human lactamase gene

<400> 67
aggtcaggat caacgagcat tctcagctgg gca 33

<210>  68
<211>  38
<212>  DNA
<213>  Artificial Sequence

<220>
<223>  Forward primer for the promoter of the human lactamase gene

<400>  68
aggtcaggat caacgtatag agcattctca gctgggcg                              38


<210>  69
<211>  34
<212>  DNA
<213>  Artificial Sequence

<220>
<223>  Reverse primer for the promoter of the human lactamase gene

<400>  69
catcttgcat gatccagggc tgctttggtt gaag                                  34


<210>  70
<211>  33
<212>  DNA
<213>  Artificial Sequence

<220>
<223>  Reverse primer for the promoter of the human lactamase gene

<400>  70
catcttgcat gatcctggca caatcttggc tca                                   33


<210>  71
<211>  38
<212>  DNA
<213>  Artificial Sequence

<220>
<223>  Forwrard primer for HLA DQA1*01 haplotype

<400>  71
acaccctgca gctgttcttc gtggcctgag ttcagcaa                              38


<210>  72
<211>  41
<212>  DNA
<213>  Artificial Sequence

<220>
<223>  Reverse primer for HLA DQA1*01 haplotype

<400>  72
gtcggaactc tgcctcttct gatgttcaag ttgtgttttg c                          41


<210>  73
<211>  39
<212>  DNA

<213>  Artificial Sequence

<220>
<223>  Forwrad primer for HLA DQA1*03 haplotype

<400>  73
acaccctgca gctgttcttc agttgcctct gttccgcag                              39


<210>  74
<211>  44
<212>  DNA
<213>  Artificial Sequence

<220>
<223>  Reverse porimer for HLA DQA1*03 haplotype

<400>  74
gtcggaactc tgcctcttct cacgatgttc aagttatgtt ttac                        44


<210>  75
<211>  39
<212>  DNA
<213>  Artificial Sequence

<220>
<223>  Forwrad primer for NGS

<400>  75
aggtcaggat caacgctcaa gttttattat aaggcctgc                              39


<210>  76
<211>  41
<212>  DNA
<213>  Artificial Sequence

<220>
<223>  Reverse primer for NGS

<400>  76
catcttgcat gatccaacct tcgtactcat gaaaatggtc a                           41


<210>  77
<211>  39
<212>  DNA
<213>  Artificial Sequence

<220>
<223>  Forwrad primer for NGS

<400>  77
aggtcaggat caacgctcaa ggtgtttctc ccttctcag                              39


<210>  78
<211>  40
<212>  DNA
<213>  Artificial Sequence

<220>

<223>  Reverse primer for NGS

<400>  78
catcttgcat gatccaacct tctttatggc aaatacacaa                          40


<210>  79
<211>  51
<212>  DNA
<213>  Artificial Sequence

<220>
<223>  Forward primer for NGS

<400>  79
cgtatcgcct ccctcgcgcc atcagacgag tgcgtaggtc aggatcaacg c             51


<210>  80
<211>  51
<212>  DNA
<213>  Artificial Sequence

<220>
<223>  Forwrad primer for NGS

<400>  80
cgtatcgcct ccctcgcgcc atcagacgct cgacaaggtc aggatcaacg c             51


<210>  81
<211>  51
<212>  DNA
<213>  Artificial Sequence

<220>
<223>  Forwrad primer for NGS

<400>  81
cgtatcgcct ccctcgcgcc atcagagacg cactcaggtc aggatcaacg c             51


<210>  82
<211>  51
<212>  DNA
<213>  Artificial Sequence

<220>
<223>  Reverse primer for NGS

<400>  82
ctatgcgcct tgccagcccg ctcagacgag tgcgtcatct tgcatgatcc a             51


<210>  83
<211>  51
<212>  DNA
<213>  Artificial Sequence

<220>
<223>  Reverse primer for NGS

<400>  83

ctatgcgcct tgccagcccg ctcagacgct cgacacatct tgcatgatcc a                51

<210>  84
<211>  51
<212>  DNA
<213>  Artificial Sequence

<220>
<223>  Reverse primer for NGS

<400>  84
ctatgcgcct tgccagcccg ctcagagacg cactccatct tgcatgatcc a                51